# EUROPEAN PATENT APPLICATION

(11) **EP 3 733 213 A1**
(43) Date of publication of application: **04.11.2020**
(21) Application number: 20158701.1
(22) Date of filing: 21.02.2020
(51) Int. Cl.: A61K 48/00, C12N 15/113

(54) **TUMOR-TARGETING TRANS-SPLICING RIBOZYME AND USE THEREOF**

(30) Priority: 22.02.2019 KR 20190021191; 20.02.2020 KR 20200021014
(71) Applicant: Rznomics Inc., Gwangju 61011 (KR)
(72) Inventor: Lee, Seong-Wook, 02780 Seoul (KR); Lee, Chang Ho, 16868 Yongin-si, Gyeonggi-do (KR); Han, Seung Ryul, 16919 Yongin-si, Gyeonggi-do (KR); Kim, Ji Hyun, 03380 Seoul (KR); Cho, Eun Yi, 16902 Yongin-si, Gyeonggi-do (KR); Jeong, Jin Sook, 48099 Busan (KR); Ju, Mi Ha, 47323 Busan (KR)
(74) Representative: Schiweck Weinzierl Koch Patentanwälte Partnerschaft mbB

(57) **Abstract**

The present invention relates to a cancer-specific trans-splicing ribozyme and a use thereof. Since a trans-splicing ribozyme according to the present invention does not act on normal tissue, but is specifically expressed in cancer tissue, it is very safe and has excellent expression efficiency at the post-transcription level, and thus can be effectively used in treatment of cancer. Specifically, as a recombinant vector of the present invention has a CMV promoter and SD/SA and WPRE sequences, the recombinant vector has high ribozyme expression efficiency, and as the recombinant vector of the present invention has miR-122T, it can regulate activity related to miR-122, and it is very safe to utilize. Therefore, the recombinant vector of the present invention can be effectively used in treatment of all types of liver cancer, except some types of liver cancer caused by HCV in which miR-122 expression is highly shown in the cancer tissue in comparison to normal tissue, and the recombinant vector also can be effectively used in treatment of other carcinomas in which miR-122 is not substantially expressed.

<110>     RZNOMICS INC.

<120>     TUMOR-TARGETING TRANS-SPLICING RIBOZYME AND USE THEREOF

<130>     EP2020002G_G20E10C0372P/EP

<150>     KR 1020190021191
<151>     2019-02-22

<150>     KR 1020200021014
<151>     2020-02-20

<160>     11

<170>     KoPatentIn 3.0

<210>     1
<211>     255
<212>     DNA
<213>     Artificial Sequence

<220>
<223>     CMV promoter sequence

<400>     1
gacattgatt attgactagt tattaatagt aatcaattac ggggtcatta gttcatagcc          60

catatatgga gttccgcgtt acataactta cggtaaatgg cccgcctggc tgaccgccca         120

acgacccccg cccattgacg tcaataatga cgtatgttcc catagtaacg ccaataggga         180

ctttccattg acgtcaatgg gtggagtatt tacggtaaac tgcccacttg gcagtacatc         240

aagtgtatca tatgc                                                          255

<210>     2
<211>     4018
<212>     DNA
<213>     Artificial Sequence

<220>
<223>     hTERT mRNA sequence

<400>     2
caggcagcgc tgcgtcctgc tgcgcacgtg ggaagccctg gccccggcca cccccgcgat          60

gccgcgcgct ccccgctgcc gagccgtgcg ctccctgctg cgcagccact accgcgaggt         120

gctgccgctg gccacgttcg tgcggcgcct ggggccccag ggctggcggc tggtgcagcg         180

cggggacccg gcggctttcc gcgcgctggt ggcccagtgc ctggtgtgcg tgccctggga         240

cgcacggccg ccccccgccg ccccctcctt ccgccaggtg tcctgcctga aggagctggt         300

ggcccgagtg ctgcagaggc tgtgcgagcg cggcgcgaag aacgtgctgg ccttcggctt         360

cgcgctgctg gacggggccc gcgggggccc ccccgaggcc ttcaccacca gcgtgcgcag         420

ctacctgccc aacacggtga ccgacgcact gcgggggagc ggggcgtggg ggctgctgct         480

gcgccgcgtg ggcgacgacg tgctggttca cctgctggca cgctgcgcgc tctttgtgct 540

ggtggctccc agctgcgcct accaggtgtg cgggccgccg ctgtaccagc tcggcgctgc 600

cactcaggcc cggcccccgc cacacgctag tggaccccga aggcgtctgg gatgcgaacg 660

ggcctggaac catagcgtca gggaggccgg ggtccccctg ggcctgccag ccccgggtgc 720

gaggaggcgc gggggcagtg ccagccgaag tctgccgttg cccaagaggc ccaggcgtgg 780

cgctgcccct gagccggagc ggacgcccgt tgggcagggg tcctgggccc acccgggcag 840

gacgcgtgga ccgagtgacc gtggtttctg tgtggtgtca cctgccagac ccgccgaaga 900

agccacctct ttggagggtg cgctctctgg cacgcgccac tcccacccat ccgtgggccg 960

ccagcaccac gcgggccccc catccacatc gcggccacca cgtccctggg acacgccttg 1020

tcccccggtg tacgccgaga ccaagcactt cctctactcc tcaggcgaca aggagcagct 1080

gcggccctcc ttcctactca gctctctgag gcccagcctg actggcgctc ggaggctcgt 1140

ggagaccatc tttctgggtt ccaggccctg gatgccaggg actccccgca ggttgccccg 1200

cctgccccag cgctactggc aaatgcggcc cctgtttctg gagctgcttg ggaaccacgc 1260

gcagtgcccc tacggggtgc tcctcaagac gcactgcccg ctgcgagctg cggtcacccc 1320

agcagccggt gtctgtgccc gggagaagcc ccagggctct gtggcggccc ccgaggagga 1380

ggacacagac ccccgtcgcc tggtgcagct gctccgccag cacagcagcc cctggcaggt 1440

gtacggcttc gtgcgggcct gcctgcgccg gctggtgccc ccaggcctct ggggctccag 1500

gcacaacgaa cgccgcttcc tcaggaacac caagaagttc atctccctgg ggaagcatgc 1560

caagctctcg ctgcaggagc tgacgtggaa gatgagcgtg cgggactgcg cttggctgcg 1620

caggagccca ggggttggct gtgttccggc cgcagagcac cgtctgcgtg aggagatcct 1680

ggccaagttc ctgcactggc tgatgagtgt gtacgtcgtc gagctgctca ggtctttctt 1740

ttatgtcacg gagaccacgt ttcaaaagaa caggctcttt ttctaccgga agagtgtctg 1800

gagcaagttg caaagcattg gaatcagaca gcacttgaag agggtgcagc tgcgggagct 1860

gtcggaagca gaggtcaggc agcatcggga agccaggccc gccctgctga cgtccagact 1920

ccgcttcatc cccaagcctg acgggctgcg gccgattgtg aacatggact acgtcgtggg 1980

agccagaacg ttccgcagag aaaagagggc cgagcgtctc acctcgaggg tgaaggcact 2040

gttcagcgtg ctcaactacg agcgggcgcg gcgccccggc ctcctgggcg cctctgtgct 2100

gggcctggac gatatccaca gggcctggcg caccttcgtg ctgcgtgtgc gggcccagga 2160

cccgccgcct gagctgtact ttgtcaaggt ggatgtgacg ggcgcgtacg acaccatccc 2220

ccaggacagg ctcacggagg tcatcgccag catcatcaaa ccccagaaca cgtactgcgt 2280

gcgtcggtat gccgtggtcc agaaggccgc ccatgggcac gtccgcaagg ccttcaagag 2340

ccacgtctct accttgacag acctccagcc gtacatgcga cagttcgtgg ctcacctgca 2400

ggagaccagc ccgctgaggg atgccgtcgt catcgagcag agctcctccc tgaatgaggc 2460

cagcagtggc ctcttcgacg tcttcctacg cttcatgtgc caccacgccg tgcgcatcag 2520

gggcaagtcc tacgtccagt gccaggggat cccgcagggc tccatcctct ccacgctgct 2580

ctgcagcctg tgctacggcg acatggagaa caagctgttt gcggggattc ggcgggacgg 2640

gctgctcctg cgtttggtgg atgatttctt gttggtgaca cctcacctca cccacgcgaa 2700

aaccttcctc aggaccctgg tccgaggtgt ccctgagtat ggctgcgtgg tgaacttgcg 2760

gaagacagtg gtgaacttcc ctgtagaaga cgaggccctg ggtggcacgg cttttgttca 2820

gatgccggcc cacggcctat tcccctggtg cggcctgctg ctggataccc ggaccctgga 2880

ggtgcagagc gactactcca gctatgcccg gacctccatc agagccagtc tcaccttcaa 2940

ccgcggcttc aaggctggga ggaacatgcg tcgcaaactc tttggggtct tgcggctgaa 3000

gtgtcacagc ctgtttctgg atttgcaggt gaacagcctc cagacggtgt gcaccaacat 3060

ctacaagatc ctcctgctgc aggcgtacag gtttcacgca tgtgtgctgc agctcccatt 3120

tcatcagcaa gtttggaaga accccacatt tttcctgcgc gtcatctctg acacggcctc 3180

cctctgctac tccatcctga aagccaagaa cgcagggatg tcgctggggg ccaagggcgc 3240

cgccggccct ctgccctccg aggccgtgca gtggctgtgc caccaagcat tcctgctcaa 3300

gctgactcga caccgtgtca cctacgtgcc actcctgggg tcactcagga cagcccagac 3360

gcagctgagt cggaagctcc cggggacgac gctgactgcc ctggaggccg cagccaaccc 3420

ggcactgccc tcagacttca agaccatcct ggactgatgg ccacccgccc acagccaggc 3480

cgagagcaga caccagcagc cctgtcacgc cgggctctac gtcccaggga gggaggggcg 3540

gcccacaccc aggcccgcac cgctgggagt ctgaggcctg agtgagtgtt tggccgaggc 3600

ctgcatgtcc ggctgaaggc tgagtgtccg gctgaggcct gagcgagtgt ccagccaagg 3660

gctgagtgtc cagcacacct gccgtcttca cttccccaca ggctggcgct cggctccacc 3720

ccagggccag cttttcctca ccaggagccc ggcttccact ccccacatag gaatagtcca 3780

tccccagatt cgccattgtt cacccctcgc cctgccctcc tttgccttcc acccccacca 3840

tccaggtgga gaccctgaga aggaccctgg gagctctggg aatttggagt gaccaaaggt 3900

gtgccctgta cacaggcgag gaccctgcac ctggatgggg gtccctgtgg gtcaaattgg 3960

ggggaggtgc tgtgggagta aaatactgaa tatatgagtt tttcagtttt gaaaaaa 4018

<210> 3
<211> 393
<212> DNA
<213> Artificial Sequence

<220>
<223> hTERT targeting trans-splicing ribozyme

<400>    3
ggcaggaaaa gttatcaggc atgcacctgg tagctagtct ttaaaccaat agattgcatc    60

ggtttaaaag gcaagaccgt caaattgcgg gaaaggggtc aacagccgtt cagtaccaag    120

tctcagggga aactttgaga tggccttgca aagggtatgg taataagctg acggacatgg    180

tcctaaccac gcagccaagt cctaagtcaa cagatcttct gttgatatgg atgcagttca    240

cagactaaat gtcggtcggg gaagatgtat tcttctcata agatatagtc ggacctctcc    300

ttaatgggag ctagcggatg aagtgatgca acactggagc cgctgggaac taatttgtat    360

gcgaaagtat attgattagt tttggagtac tcg                                 393

<210>    4
<211>    1131
<212>    DNA
<213>    Artificial Sequence

<220>
<223>    Hsv-tk

<400>    4
atggcttcgt acccctgcca tcaacacgcg tctgcgttcg accaggctgc gcgttctcgc    60

ggccatagca accgacgtac ggcgttgcgc cctcgccggc agcaagaagc cacggaagtc    120

cgcctggagc agaaaatgcc cacgctactg cgggtttata tagacggtcc tcacgggatg    180

gggaaaacca ccaccacgca actgctggtg gccctgggtt cgcgcgacga tatcgtctac    240

gtacccgagc cgatgactta ctggcaggtg ctgggggctt ccgagacaat cgcgaacatc    300

tacaccacac aacaccgcct cgaccagggt gagatatcgg ccggggacgc ggcggtggta    360

atgacaagcg cccagataac aatgggcatg ccttatgccg tgaccgacgc cgttctggct    420

cctcatatcg ggggggaggc tgggagctca catgccccgc ccccggccct caccctcatc    480

ttcgaccgcc atcccatcgc cgccctcctg tgctacccgg ccgcgcgata ccttatgggc    540

agcatgaccc cccaggccgt gctggcgttc gtggccctca tcccgccgac cttgcccggc    600

acaaacatcg tgttgggggc ccttccggag gacagacaca tcgaccgcct ggccaaacgc    660

cagcgccccg gcgagcggct tgacctggct atgctggccg cgattcgccg cgtttacggg    720

ctgcttgcca atacggtgcg gtatctgcag ggcggcgggt cgtggcggga ggattgggga    780

cagctttcgg ggacggccgt gccgccccag ggtgccgagc cccagagcaa cgcgggccca    840

cgaccccata tcggggacac gttatttacc ctgtttcggg cccccgagtt gctggccccc    900

aacggcgacc tgtacaacgt gtttgcctgg gccttggacg tcttggccaa acgcctccgt    960

cccatgcacg tctttatcct ggattacgac caatcgcccg ccggctgccg ggacgccctg    1020

ctgcaactta cctccgggat ggtccagacc cacgtcacca cccccggctc cataccgacg    1080

atctgcgacc tggcgcgcac gtttgcccgg gagatggggg aggctaactg a 1131

<210> 5
<211> 22
<212> DNA
<213> Artificial Sequence

<220>
<223> miR-122T

<400> 5
caaacaccat tgtcacactc ca 22

<210> 6
<211> 181
<212> DNA
<213> Artificial Sequence

<220>
<223> SD/SA sequence

<400> 6
agatctgaac tgaaaaacca gaaagttaac tggtaagttt agtctttttg tcttttattt 60

caggtcccgg atccggtggt ggtgcaaatc aaagaactgc tcctcagtgg atgttgcctt 120

tacttctagg cctgtacgga agtgttactt ctgctctaaa agctgcggaa ttgtacccag 180

g 181

<210> 7
<211> 589
<212> DNA
<213> Artificial Sequence

<220>
<223> WPRE sequence

<400> 7
aatcaacctc tggattacaa aatttgtgaa agattgactg gtattcttaa ctatgttgct 60

ccttttacgc tatgtggata cgctgcttta atgcctttgt atcatgctat tgcttcccgt 120

atggctttca ttttctcctc cttgtataaa tcctggttgc tgtctcttta tgaggagttg 180

tggcccgttg tcaggcaacg tggcgtggtg tgcactgtgt ttgctgacgc aacccccact 240

ggttggggca ttgccaccac ctgtcagctc ctttccggga ctttcgcttt ccccctccct 300

attgccacgg cggaactcat cgccgcctgc cttgcccgct gctggacagg ggctcggctg 360

ttgggcactg acaattccgt ggtgttgtcg gggaaatcat cgtcctttcc ttggctgctc 420

gcctgtgttg ccacctggat tctgcgcggg acgtccttct gctacgtccc ttcggccctc 480

aatccagcgg accttccttc ccgcggcctg ctgccggctc tgcggcctct tccgcgtctt 540

cgccttcgcc ctcagacgag tcggatctcc ctttgggccg cctccccgc                589

<210>    8
<211>    326
<212>    DNA
<213>    Artificial Sequence

<220>
<223>    ribozyme antisense sequence

<400>    8
aaggccagca cgttcttcgc gccgcgctcg cacagcctct gcagcactcg ggccaccagc      60

tccttcaggc aggacacctg gcggaaggag ggggcggcgg ggggcggccg tgcgtcccag     120

ggcacgcaca ccaggcactg ggccaccagc gcgcggaaag ccgccgggtc cccgcgctgc     180

accagccgcc agccctgggg ccccaggcgc cgcacgaacg tggccagcgg cagcacctcg     240

cggtagtggc tgcgcagcag ggagcgcacg gctcggcagc ggggagcgcg cggcatcgcg     300

ggggtggccg gggccagggc ttccca                                          326

<210>    9
<211>    56
<212>    DNA
<213>    Artificial Sequence

<220>
<223>    Beta-globin poly(A) signal

<400>    9
aataaaggaa atttattttc attgcaatag tgtgttggaa ttttttgtgt ctctca          56

<210>    10
<211>    22
<212>    DNA
<213>    Artificial Sequence

<220>
<223>    ribozyme forward primer

<400>    10
ttccggagga cagacacatc ga                                               22

<210>    11
<211>    22
<212>    DNA
<213>    Artificial Sequence

<220>
<223>    ribozyme reverse primer

<400>    11

gcagataccg caccgtattg gc 22

## Description

### [FIELD OF THE INVENTION]

The present invention relates to a cancer-specific trans-splicing ribozyme and a use thereof.

### [DISCUSSION OF RELATED ART]

Telomerase is a ribonucleoprotein, which enables immortal proliferation of cells by reversing a shortening region of telomere during DNA replication by repeatedly adding a TTAGGG sequence to the end of a telomere present at the 3' end of a chromosome. Telomerase is one of the most important enzymes among those regulating the immortalization and proliferation ability of cancer cells. While hematopoietic cells and about 80 to 90% of cancer cells possess telomerase activity, normal cells neighboring cancer cells do not exhibit the activity. Moreover, telomerase reactivation has a major effect on immortal growths of advanced metastatic cancer.

Human telomerase consists of two components including human telomerase RNA (hTR), which acts as substrate RNA and human telomerase reverse transcriptase (hTERT) which serves as a catalyst. hTERT genes are expressed in proportion to telomerase activity, and there is a strong correlation between an intracellular hTERT level and cellular telomerase activity. Particularly, TERT activity can be observed in about 90% or more of cancer patients.

Recently, trans-splicing ribozymes targeting the hTERT has been reported, and its possible use as a cancer therapeutic agent has been investigated. However, while a combination of a trans-splicing ribozyme and a tissue-specific promoter shows a high tissue specificity, their expression efficiency is very low, making its use for therapeutic purpose as unsatisfactory. In addition, since telomerase is also active in certain normal cells such as stem cells, hematopoietic stem cells, germ cells, and regenerating normal hepatocytes, hTERT-targeting treatment can have toxic effects towards these normal cells. It is reported that about 5% of normal hepatocytes possess weak telomerase activity, and the telomerase activity increases in regenerating liver. Particularly, a majority of hepatocellular carcinoma (HCC) is accompanied by liver cirrhosis, and non-tumorous hepatocytes in a regenerative nodule in the liver cirrhosis region exhibit TERT expression at a low level.

Korean Patent Application Publication No. 10-2016-0038674 A, describes a recombinant vector which includes a ribozyme-target gene expression cassette that includes a tissue-specific promoter, a trans-splicing ribozyme targeting a cancer-specific gene, and a target gene connected to the 3' exon of the ribozyme, and additionally a nucleic acid sequence recognizing microRNA-122 (miR-122). It also describes a use of the ribozyme expressed therefrom for preventing or treating liver cancer. MiR-122 is microRNA known to be very highly expressed in a normal liver, but less expressed in advanced liver cancer cells. According to prior art, based on the above-mentioned phenomenon, as a miR-122 targeting region is introduced to the 3' UTR region of a ribozyme expression vector, a ribozyme delivered to the liver is not expressed in a normal liver due to overexpressed miR-122 but is expressed in liver cancer cells having a decreased miR-122 level.

However, the above-discussed conventional approach has a drawback that a high count of vector-introduced viruses is needed to exhibit a therapeutic effect. In addition, it has been reported that an increased expression of miR-122 was found frequently in a liver tissue with cancer caused by hepatitis C virus (HCV) infection, compared to a normal liver tissue. For this reason, effectiveness of the above discussed conventional approach to treat liver cancer where the expression of miR-122 increases or other cancers is questionable.

### [PRIOR ART REFERENCES]

### [PATENT LITERATURE]

(Patent Literature 1) KR Patent Application Publication No. 10-2016-0038674A

### [DESCRIPTION OF INVENTION]

### [PROBLEM TO SOLVE]

To solve the above-described problem, the present invention is directed to providing a cancer-specific trans-splicing ribozyme possessing an excellent safety and expression efficiency, suitable for treating cancer.

### [SOLUTION TO SOLVE THE PROBLEM]

The present invention provides a recombinant vector, which includes:
(i) a cytomegalovirus (CMV) promoter;
(ii) a ribozyme-desired gene expression cassette, which includes a sequence encoding a trans-splicing ribozyme targeting a cancer-specific gene and a desired gene connected to a 3' exon of the ribozyme-encoding sequence,
   in which the expression cassette has a splicing donor/splicing acceptor sequence (SD/SA sequence) connected to the 5' end of the ribozyme-desired gene expression cassette, and a Woodchuck hepatitis virus posttranscriptional regulatory element (WPRE) connected to the 3' end of the ribozyme-desired gene expression cassette; and
(iii) a nucleic acid sequence recognizing microRNA-122 (miR-122), which is connected to the 3' end of the WPRE.

In addition, the present invention provides a gene delivery system, which includes the recombinant vector.

Moreover, the present invention provides a ribozyme expressed from the recombinant vector.

Furthermore, the present invention provides a pharmaceutical composition for preventing or treating cancer, which includes the recombinant vector, a gene delivery system or a ribozyme as an active ingredient.

### [ADVANTAGEOUS EFFECTS]

A trans-splicing ribozyme according to the present invention does not act on normal tissue, but is specifically expressed in cancer tissue, it is very safe and has excellent expression efficiency at the post-transcription level, and thus can be effectively used in treatment of cancer. Specifically, as a recombinant vector of the present invention has a CMV promoter and SD/SA and WPRE sequences, the recombinant vector has high ribozyme expression efficiency, and as the recombinant vector of the present invention has miR-122T, it can regulate activity related to miR-122, and it is very safe to utilize. Therefore, the recombinant vector of the present invention can be effectively used in treatment of all types of liver cancer, except some types of liver cancer caused by HCV in which miR-122 expression is highly shown in the cancer tissue in comparison to normal tissue, and the recombinant vector also can be effectively used in treatment of other carcinomas in which miR-122 is not substantially expressed.

### [BRIEF DESCRIPTION OF THE DRAWINGS]

FIG. 1 is a schematic diagram of the configuration of an expression cassette for a CMV promoter-based hTERT-targeting trans-splicing ribozyme and a target gene according to the present invention;
FIG. 2 is a set of graphs showing cell survival rates according to the MOI of ECRT-122T adenovirus transduced into (a) a Hep3B cell line not expressing miR-122 and (b) a Huh-7.5 cell line expressing miR-122, respectively;
FIG. 3 is a set of graphs showing cell survival rates according to the MOI of ECRT-122T adenovirus transduced into cell lines not expressing miR-122 (a: Hep3B, b: SNU398, c: SNU449);
FIG. 4 is a set of graphs showing cell survival rates according to the MOI of ECRT-122T adenovirus transduced into miR-122-expressing cell lines (a: Huh-7, b: Huh-7.5);
FIG. 5 is a set of graphs showing cell survival rates according to the MOI of ECRT-122T adenovirus transduced into various types of liver cancer cell lines expressing or not expressing miR-122;
FIG. 6 is a set of graphs showing cell survival rates according to the MOI of ECRT-122T adenovirus transduced into cell lines derived from liver cancer patients sensitized or not sensitized to sorafenib;
FIG. 7 show the results of identification of (a) a tumor weight, (b) tumor tissue and (c) liver enzyme levels (glutamic oxaloacetic transaminase (GOT) and glutamic pyruvic transaminase (GPT)) according to an injection dose of an adenovirus expressing ECRT or ECRT-122T in animal liver cancer models;
FIG. 8 is a set of graphs showing RNA expression levels (a: E4, b: ribozyme, c: miR-122) according to the MOI of recombinant vectors transduced into Huh-7 cell lines expressing miR-122;
FIG. 9 is a set of graphs showing RNA expression levels (a: E4, b: ribozyme, c: miR-122) according to the MOI of an ECRT-122T adenovirus transduced into Huh-7.5 cell lines expressing miR-122;
FIG. 10 is a set of graphs showing cell survival rates, miR-122 expression levels and ribozyme expression levels according to the level of concentration of tetracycline and MOI of ECRT-122T adenovirus transduced into Hep3B-stabilized cell line clones having an miR-122 tetracycline-on system (#7-14, 7-4 and 7-2);
FIG. 11 is a set of graphs comparing miR-122 expression levels in normal liver tissue and liver cancer tissue of liver cancer patients (a: entire patient group, b: HBV-associated patient group, c: HCV-associated patient group, d: alcohol-associated patient group, e: chronic hepatitis-associated patient group excluding HBV and HCV-associated patient groups, f: patient group associated with other etiological factors);
FIG. 12 is a set of graphs showing analyses of (a) mRNA and (b) protein levels of hTERT and (c) miR-122 levels in an established liver cancer cell line derived from the liver cancer cells of patients, respectively;
FIG. 13 is a set of graphs showing cell survival rates according to the MOI of ECRT-122T adenovirus transduced into a colon cancer cell line, a glioblastoma cell line, a melanoma cell line, a cervical cancer cell line, a lung cancer cell line, an osteosarcoma cell line and a breast cancer cell line;
FIG. 14 is a set of graphs showing cell survival rates according to the MOI of ECRT-122T adenovirus transduced into bile duct cancer cell lines (a: SNU478, b: SNU869);
FIG. 15 is a graph identifying (a) a tumor size and (b) a tumor weight as a result of injecting glioblastoma cells, that is, a LN229 cell line, into a nude mouse to induce tumorigenesis, and then administering ECRT-122T adenovirus;
FIG. 16 is a graph identifying a tumor size as a result of injecting glioblastoma cells, that is, a U87MG cell line, into a nude mouse to induce tumorigenesis, and then administering ECRT-122T adenovirus;
FIG. 17 is a graph showing the degree of distribution of a recombinant vector in major tissue after ECRT-122T adenovirus is injected into a normal ICR mouse through an intravenous injection;
FIG. 18 is a graph showing the degree of distribution of a recombinant vector in major tissue after ECRT-122T adenovirus is injected into a rat through a hepatic arterial injection;
FIG. 19 is a graph showing the results of quantifying HSVtk DNA by isolating genomic DNA (gDNA) from each organ after ECRT-122T adenovirus is injected into a rat through a hepatic arterial injection;
FIG. 20 is a graph showing the results of confirming ribozyme expression levels from RNA isolated from the liver after ECRT-122T adenovirus is injected into a rat through a hepatic arterial injection;
FIG. 21 is a graph showing the results of measuring AST and ALT levels after ECRT-122T adenovirus is injected into a normal ICR mouse and then treated with GCV;
FIG. 22 is a graph showing the results of measuring (a) the body weight, (b) the feed consumption and (c) the liver weight of a mouse after ECRT-122T adenovirus is injected into a normal ICR mouse and then treated with GCV;
FIG. 23 shows the histopathological examination results for the liver after different doses of the ECRT-122T adenovirus are injected into a normal ICR mouse
FIG. 24 is a set of graphs showing the results of measuring AST and ALT levels after ECRT-122T adenovirus is injected into a normal ICR mouse;
FIG. 25 is a set of graphs showing the results of measuring (a) the body weight, (b) the feed consumption and (c) the liver weight of a mouse after ECRT-122T adenovirus is injected into a normal ICR mouse;
FIG. 26 shows the histopathological examination results for the liver after different doses of the ECRT-122T adenovirus were injected into a normal ICR mouse and then treated with GCV;
FIG. 27 shows the results of comparing anticancer efficacy as a result of injecting Hep3B cells into a mouse to induce liver cancer formation, and then administering CRT-122T or ECRT-122T adenovirus;
FIG. 28 shows the results of comparing anticancer efficacy as a result of injecting SNU398 cells into xenograft subcutaneous models of mice to induce tumorigenesis, and then administering CRT-122T or ECRT-122T adenovirus; and
FIG. 29 is a graph showing the results of measuring AST and ALT levels after ECRT or ECRT-122T adenovirus is injected into a normal ICR mouse.

### DETAILED DESCRIPTION OF EXEMPLARY EMBODIMENTS

Hereinafter, the present invention will be described in further detail.

In the present invention, when a part "includes" one component, it means that, unless particularly stated otherwise, another component may be further included, not excluding the relevant component.

### Recombinant vector

One aspect of the present invention provides a recombinant vector, which includes:
(i) a cytomegalovirus (CMV) promoter;
(ii) a ribozyme-desired gene expression cassette, which includes a trans-splicing ribozyme-encoding sequence and a desired gene connected to a 3' exon of the ribozyme, said ribozyme targeting a cancer specific gene,
   in which the expression cassette has a SD/SA sequence connected to the 5' end of the ribozyme-desired gene expression cassette, and WPRE connected to the 3' end of the ribozyme-desired gene expression cassette; and
(iii) an additional nucleic acid sequence recognizing microRNA-122 (miR-122), which is connected to the 3' end of the WPRE.

Based on the findings that a recombinant vector has an excellent cancer treating effect *in vivo* when including a cytomegalovirus (CMV) promoter, a ribozyme, a SD/SA sequence and WPRE, which is connected at both ends of a desired gene, as constituents, and as the recombinant vector employs a CMV promoter, a SD/SA sequence and WPRE at the same time and further includes a nucleic acid sequence recognizing miR-122, various cancer cells as well as liver cancer cells can be killed.

The term "vector" used herein is an expression vector capable of expressing a target gene within suitable host cells and a gene construct including an essential regulatory element operably linked in order to express a gene insert included in the vector.

The term "operably linked" used herein refers to a functional linkage between a nucleic acid expression regulating sequence performing a general function and a nucleic acid sequence encoding a target gene.

For example, when a ribozyme-encoding sequence is operably linked to a promoter, the expression of the ribozyme-encoding sequence will be under the influence or control of the promoter. Two nucleic acid sequences (the ribozyme-encoding sequence and a promoter region sequence at the 5' end of the ribozyme-encoding sequence) are operably linked when promoter activity is induced and the ribozyme-encoding sequence is then transcribed, and it may be considered that the two nucleic acid sequences are operably linked when the linkage properties between the two sequences do not induce a frame shift mutation, and the expression regulating sequence does not inhibit ribozyme expression. The operable linkage to the recombinant vector may be formed using a gene recombination technique well known in the art, and site-specific DNA cleavage and linkage may use an enzyme generally known in the art.

The vector according to the present invention may include a signal sequence or leader sequence for membrane targeting or secretion as well as expression regulatory factors such as a promoter, an operator, an initiation codon, a termination codon, a polyadenylation signal and an enhancer, and may be manufactured in various ways according to an intended purpose. A promoter of the vector may be a constitutive or inducible promoter. In addition, the expression vector may include a selectable marker for selecting host cells containing a vector, and in the case of a replicable expression vector, an origin of replication may be included. The vector may be self-replicated or integrated into host DNA.

The vector according to the present invention is preferably a plasmid vector, a cosmid vector or a virus vector, and most preferably, a virus vector. The virus vector is preferably derived from a retrovirus, such as human immunodeficiency virus (HIV), murine leukemia virus (MLV), avian sarcoma/leucosis virus (ASLV), spleen necrosis virus (SNV), Rous sarcoma virus (RSV) or mouse mammary tumor virus (MMTV), an adenovirus, an adeno-associated virus (AAV), or a herpes simplex virus (HSV), but the present invention is not limited thereto. The recombinant vector according to the present invention is most preferably a recombinant adenovirus vector.

The term "expression cassette" used herein means a unit cassette capable of expressing a trans-splicing ribozyme-desired gene, which includes a CMV promoter and a trans-splicing ribozyme-desired gene, has a SD/SA sequence and a WPRE sequence at both ends of the trans-splicing ribozyme-desired gene, and a nucleic acid sequence recognizing miR-122 additionally connected to the 3' end of the WPRE.

The trans-splicing ribozyme-desired gene expression cassette according to the present invention may further include an expression regulation inducer to regulate a transcription level. In the present invention, particularly, a SD/SA sequence and/or WPRE is(are) linked, and a sequence recognizing miR-122 is further linked to the 3' end of the WPRE, but the present invention is not limited thereto. To this end, an expression level of the ribozyme-desired gene may be regulated, and the ribozyme is expressed only when miR-122 is expressed at a certain level or less, such that the influence on normal hepatocytes may be minimized.

In the ribozyme-desired gene expression cassette according to the present invention, preferably, a SD/SA sequence (SD/SA) is linked to the 5' end of the ribozyme, WPRE is linked to the 3' end of the desired gene, and a sequence recognizing miR-122 is linked to the 3' end of the WPRE.

The SD/SA sequence according to the present invention may increase transcription initiation, processing of RNA polymerase II and the nucleocytoplasmic export of mRNA, and the WPRE according to the present invention may increase processing and nucleocytoplasmic export of mRNA, and thus the pre-mRNA level may increase. Due to the configuration described above, the RNA level of a ribozyme in cells is significantly increased to allow the death of cancer cells *in vivo* to increase and allow cancer cell-specific expression, resulting in the reduction in toxicity towards normal cells.

The SD/SA sequence according to the present invention is a sequence corresponding to the starting region/ending region of an intron cleaved by splicing to remove an intron of a RNA transcript, and generally, the SD sequence may be a GU sequence at the 5' end of the intron, and the SA sequence may be an AG sequence at the 3' end of the intron.

The WPRE according to the present invention refers to a sequence increasing gene expression as a result of inducing a tertiary structure that promotes transcription in DNA.

In the present invention, the SD/SA sequence and WPRE sequence may include sequences of SEQ ID NO: 6 and SEQ ID NO: 7, respectively, but are not limited as long as they are present in the desired gene expression cassette and promote the expression of a desired gene.

The nucleic acid sequence recognizing miR-122 according to the present invention is called a microRNA-122 target site (miR-122T) in the specification. The miR-122T may include one or more, for example, 1 to 10, preferably, 1 to 5, and more preferably, 1 to 3 sequences of SEQ ID NO: 5. The miR-122 is normally expressed in normal hepatocytes, but an expression level thereof is reduced in liver cancer cells. A therapeutic agent with increased sensitivity and specificity towards liver cancer cells may be developed using the miR-122T, and in the present invention, a liver cancer cell-specific ribozyme may be expressed by including a nucleic acid sequence recognizing miR-122 to the ribozyme-encoding sequence-desired gene expression cassette.

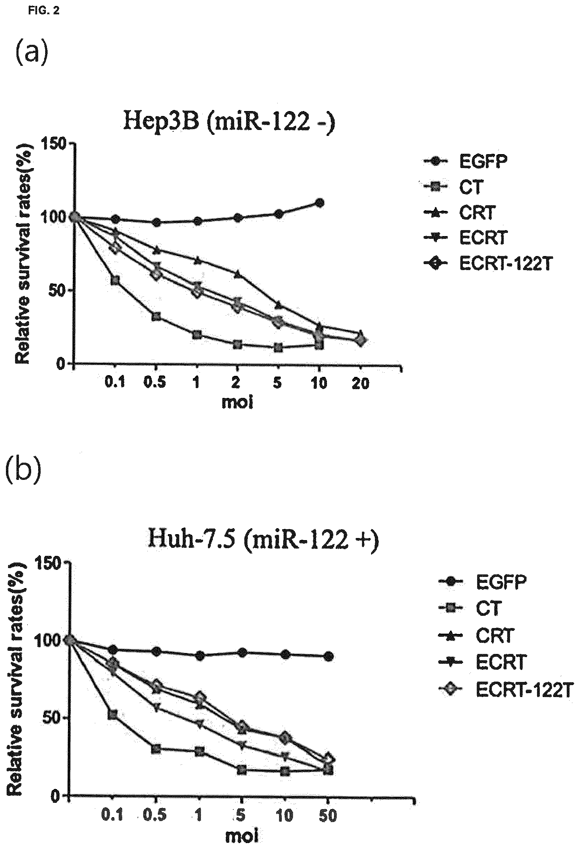

In one embodiment of the present invention, when SD/SA and WPRE are included in the expression cassette, ribozyme expression increases and thus an effect of inducing cell death further increases. In addition, miR-122T targeting miR-122 induces cell death only in liver cancer cells in which miR-122 expression decreases and does not induce cell death in normal hepatocytes in which the miR-122 expression is normally performed, and thus it was confirmed that liver cancer cell-specific treatment is possible (FIG. 2).

The term "cancer-specific gene" used herein refers to a gene specifically expressed or significantly over-expressed only in cancer cells. The cancer-specific gene may impart a characteristic by which the ribozyme according to the present invention may act cancer-specifically. Such a cancer-specific gene has preferably a telomerase reverse transcriptase (TERT) mRNA sequence, an alphafetoprotein (AFP) mRNA sequence, a carcinoembryonic antigen (CEA) mRNA sequence, a prostate-specific antigen (PSA) mRNA sequence, a cytoskeleton-associated protein 2 (CKAP2) mRNA sequence or a mutant rat sarcoma (RAS) mRNA sequence, more preferably, a telomerase reverse transcriptase (TERT) mRNA sequence, and most preferably, a human telomerase reverse transcriptase (hTERT) mRNA sequence.

The term "TERT" used herein is one of important enzymes regulating immortality and a proliferation of cancer cells, and it refers to an enzyme serving to protect a chromosomal end by forming a telomere structure on a chromosome, thereby inhibiting cell aging. In normal cells, the telomere length gradually decreases every time the cells divide, resulting in the loss of genetic material and cell death. However, in cancer cells, since such an enzyme allows the telomere to extend continuously, the cells do not die, and the enzyme directly contributes to the immortality of cancer cells and thus is known as a critical obstacle in treating cancer. In the present invention, hTERT mRNA including a sequence of SEQ ID NO: 2 may be used as a cancer-specific gene, but the present invention is not limited thereto.

The term "promoter" used herein is a part of DNA, which is involved in binding of an RNA polymerase in order to initiate transcription. Generally, the promoter is located adjacent to a desired gene as well as at an upper region thereof, and as a site to which a transcription factor which is an RNA polymerase or a protein inducing an RNA polymerase binds, the enzyme or protein can be induced to be located at a proper transcription start region. That is, the promoter includes a specific gene sequence which is located at the 5' region of a gene to be transcribed in a sense strand and induces the initiation of mRNA synthesis for a desired gene when an RNA polymerase binds to a corresponding site directly or via a transcription factor.

The promoter according to the present invention includes preferably a CMV promoter including a sequence of SEQ ID NO: 1 to increase the expression of a gene.

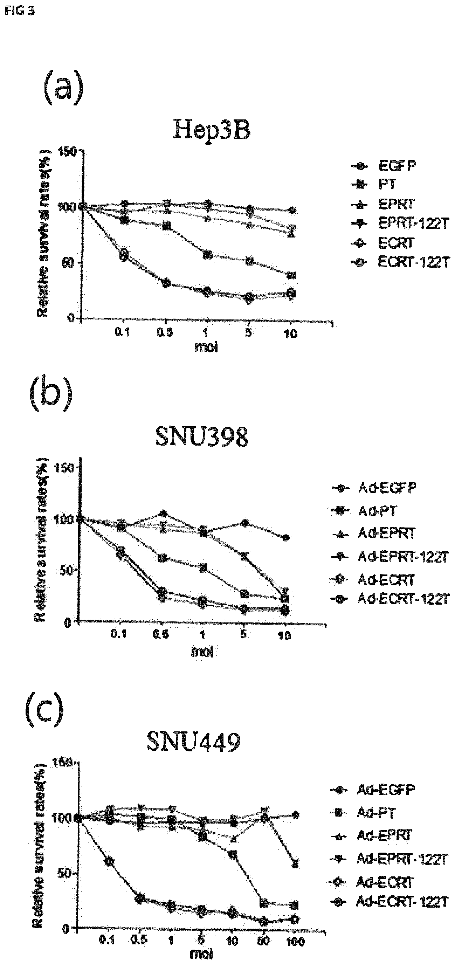

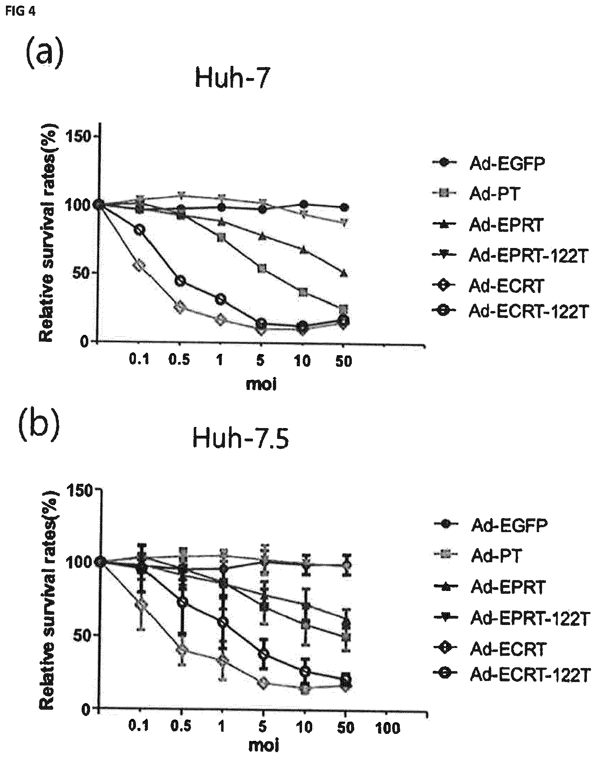

In one embodiment of the present invention, it was confirmed that when a CMV promoter is introduced into the recombinant expression vector according to the present invention, excellent ribozyme expression efficiency is achieved, and despite of existence of regulation by miR-122 due to the miR-122T contained in the recombinant vector, the high expression of ribozyme induces cell death and exhibits an anticancer effect (FIGS. 3 and 4).

The term "ribozyme" used herein is an RNA molecule acting as an enzyme or a protein molecule comprising the RNA molecule, and it is also called an RNA enzyme or catalytic RNA. The ribozyme is an RNA molecule having a three-dimensional structure, which performs a chemical reaction and has a property of self-clearing or cleaving a target RNA. It is known that some ribozymes cleave themselves or a target RNA molecule to inhibit activity of the target RNA and that other ribozymes catalyze the aminotransferase activity of a ribosome. Such ribozymes may include a hammerhead ribozyme, a VS ribozyme and a hairpin ribozyme.

The ribozyme according to the present invention may not only exhibit a selective anticancer effect by inhibiting the activity of a cancer-specific gene through a trans-splicing reaction, but also activate an anti-cancer therapeutic gene which can be co-expressed. Accordingly, any type of ribozyme can be used as long as it can inactivate a cancer-specific gene and activate an anti-cancer therapeutic gene.

The ribozyme according to the present invention is preferably a ribozyme targeting the hTERT mRNA described above, and may inhibit expression of hTERT by specifically cleaving hTERT mRNA in a cancer cell overexpressing hTERT and may specifically serve to express a therapeutic gene.

The term "trans-splicing" used herein refers to linking RNAs derived from different genes to each other. Preferably, an hTERT-targeting trans-splicing group I ribozyme whose trans-splicing ability is verified as a result of recognizing mRNA of cancer-specific hTERT may be used.

The term "desired gene" used herein refers to a gene to be linked to an mRNA of a cancer-specific gene by an action of the ribozyme and be induced to be expressed.

The desired gene according to the present invention is preferably an anti-cancer therapeutic gene or a reporter gene, and most preferably, an anti-cancer therapeutic gene.

The term "anti-cancer therapeutic gene" used herein refers to a polynucleotide sequence encoding a polypeptide exhibiting a therapeutic effect in regards to the expression in cancer cells. The anti-cancer therapeutic gene may be expressed while being conjugated with the ribozyme, or independently expressed, thereby exhibiting anticancer activity. The anti-cancer therapeutic gene is preferably one or more selected from the group consisting of a drug-sensitizing gene, a proapoptotic gene, a cytostatic gene, a cytotoxic gene, a tumor suppressor gene, an antigenic gene, a cytokine gene and an anti-angiogenic gene, and most preferably, a drug-sensitizing gene.

In the present invention, one or more anti-cancer therapeutic genes may be used individually or in combination.

The drug-sensitizing gene according to the present invention is a gene encoding an enzyme that converts a non-toxic prodrug into a toxic material, and since cells into which the gene is introduced die, it is also called a suicide gene. That is, when a non-toxic prodrug is systemically administered to normal cells, the prodrug is converted into a toxic metabolite only in cancer cells to change sensitization to a drug, resulting in the destruction of cancer cells. Such a drug-sensitizing gene is preferably a herpes simplex virus-thymidine kinase (HSVtk) gene using ganciclovir as a prodrug, or a cytosine deaminase (CD) gene of *E. coli* using 5-fluorocytosine (5-FC) as a prodrug, and most preferably, a HSVtk gene including a sequence of SEQ ID NO: 4.

The proapoptotic gene according to the present invention refers to a nucleotide sequence inducing programmed apoptosis when expressed. Examples of proapoptotic genes known to those of ordinary skill in the art may include a p53 gene, an adenovirus E3-11.6K gene (derived from Ad2 and Ad5), an adenovirus E3-10.5K gene (derived from Ad), an adenovirus E4 gene, a p53 pathway gene and a gene encoding a caspase.

The cytostatic gene according to the present invention refers to a nucleotide sequence expressed in cells to suspend a cell cycle during the cell cycle. Examples of the cytostatic genes include a p21 gene, a retinoblastoma gene, an E2F-Rb fusion protein gene, genes encoding a cyclin-dependent kinase inhibitor (e.g., p16, p15, p18 and p19) and a growth arrest specific homeobox (GAX) gene, but the present invention is not limited thereto.

The cytotoxic gene according to the present invention refers to a nucleotide sequence expressed in cells to exhibit toxicity. For example, the cytotoxic gene includes a nucleotide sequence encoding a Pseudomonas exotoxin, a lysine toxin or a Diphtheriae toxin, but the present invention is not limited thereto.

The tumor suppressor gene according to the present invention refers to a nucleotide sequence which may be expressed in target cells to suppress a tumor phenotype or induce cell death. As a representative example, the tumor suppressor gene may be a tumor necrosis factor-a (TNF-α) gene, a p53 gene, an APC gene, a DPC-4/Smad4 gene, a BRCA-1 gene, a BRCA-2 gene, a WT-1 gene, a retinoblastoma gene, an MMAC-1 gene, an adenomatous polyposis coil protein, a deleted colon carcinoma (DCC) gene, an MMSC-2 gene, an NF-1 gene, a nasopharyngeal tumor suppressor gene located on chromosome 3p21.3, an MTS1 gene, a CDK4 gene, an NF-1 gene, an NF-2 gene, a VHL gene or a programmed death-1 (sPD-1) gene.

The antigenic gene according to the present invention refers to a nucleotide sequence that is expressed in target cells to produce a cell surface antigenic protein recognized by the immune system. As examples of the antigenic gene known to those of ordinary skill in the art, the antigenic gene may include a carcinoembryonic antigen (CEA) and p53.

The cytokine gene according to the present invention refers to a nucleotide sequence that is expressed in cells to produce a cytokine. Representative examples of the cytokine genes may include GMCSF, interleukins (IL-1, IL-2, IL-4, IL-12, IL-10, IL-19, and IL-20), interferons α, β and γ (interferon α-2b) and fusions such as interferon α-2α-1.

The anti-angiogenic gene according to the present invention refers to a nucleotide sequence that is expressed to release an anti-angiogenic factor out of cells. Examples of the anti-angiogenic genes may include angiostatin, a vascular endothelial growth factor (VEGF) suppressor, and endostatin.

The term "herpes simplex virus-thymidine kinase (HSV-tk)" used herein refers to a thymidine kinase derived from a herpes simplex virus. This enzyme is a representative example of a drug-sensitizing gene that converts a non-toxic prodrug into a toxic material, leading to death of the gene-inserted cells. In the present invention, the HSV-tk gene may be used as an anti-cancer therapeutic gene that is expressed as a conjugate (or complex) with the ribozyme according to the present invention to exhibit anticancer activity. The HSVtk gene may be exemplified by GenBank Accession No. AAP13943, P03176, AAA45811, P04407, Q9QNF7, KIBET3, P17402, P06478, P06479, AAB30917, P08333, BAB84107, AAP13885, AAL73990, AAG40842, BAB11942, NP_044624, NP_044492, or CAB06747.

The term "reporter gene" used herein is a gene used to monitor whether a recombinant vector according to an example of the present invention is introduced or to monitor expression efficiency of a ribozyme, and may be any gene that enables monitoring without damage to infected cells or tissue without limitation. Preferably, the reporter gene is luciferase, a green fluorescent protein (GFP), a modified green fluorescent protein (mGFP), an enhanced green fluorescent protein (EGFP), a red fluorescent protein (RFP), a modified red fluorescent protein (mRFP), an enhanced red fluorescent protein (ERFP), a blue fluorescent protein (BFP), an enhanced blue fluorescent protein (EBFP), a yellow fluorescent protein (YFP), an enhanced yellow fluorescent protein (EYFP), a cyan fluorescent protein (CFP) or an enhanced cyan fluorescent protein (ECFP).

The expression of a cancer cell-specific ribozyme may be observed by using a reporter gene as a desired gene. The ribozyme-expression vector of the present invention includes a promoter and a miRNA target site, and therefore may specifically expressed in cancer cells, but not in normal cells. It will be apparent to those of ordinary skill in the art that the above-described method can be applied to diagnose whether cancer occurs in specific tissue.

### Gene delivery system

Another aspect of the present invention provides a gene delivery system including the recombinant vector according to the present invention.

The term "gene delivery system" used herein refers to a system that can increase expression efficiency by increasing intracellular delivery efficiency for a target gene and/or nucleic acid sequence, and may be classified into a virus-mediated system and a non-viral system.

The virus-mediated system uses a viral vector such as a retrovirus vector or an adenovirus vector, and is known to have relatively higher intracellular gene delivery efficiency than a non-viral system since it uses a virus's inherent cellular invasion mechanism that infects human cells. In addition, after entering cells, a non-viral vector has a drawback of gene degradations in an endolysosome after the endosome is fused with a lysosome, whereas a viral vector has an advantage of high gene delivery efficiency because of less gene loss thanks to a mechanism of delivering a gene into a nucleus without passing through a lysosome.

The viral vector that can be used in the present invention may be a vector derived from a retrovirus, an adenovirus or an adeno-associated virus as described in the disclosure on the recombinant vector. The viral vector may be introduced into cells by a transduction method, such as infection, after assembly into a viral particle.

In one embodiment of the present invention, a recombinant adenovirus including the above-described recombinant vector was designed as a gene delivery system. That is, the recombinant adenovirus serves to deliver a recombinant vector expressing a trans-splicing ribozyme specific to a cancer-specific gene to target cells (e.g., cancer cells), and the recombinant vector delivered into the cells is expressed by an intracellular transcription system. The expressed trans-splicing ribozyme may insert a desired gene that is linked to the ribozyme-encoding gene into the cleaved transcript of the cancer-specific gene that is abundant in cancer cells.

The non-viral system is a method of using a cationic lipid delivery system or cationic polymer delivery system as a delivery vehicle for a nucleic acid and/or a gene, or a method of using electroporation.

A cationic lipid delivery system is a method of forming a complex using a positive charge of a nanometer-sized liposome mainly consisting of a cationic lipid or nanoparticles made of a lipid with a negatively charges gene, and an expression vector or nucleic acid containing the gene, and then delivering the complex into cells by endocytosis. The complex delivered into cells is first delivered to a lysosome from an endosome, exported through the cytoplasm and then expressed. A cationic polymer delivery system delivers a gene in a manner similar to the cationic lipid delivery system, except that a polymer is used instead of a lipid, and representative cationic polymers include polyethyleneimine, poly-L-lysine and chitosan.

Accordingly, the recombinant vector of the present invention may be combined with a cationic lipid delivery system or cationic polymer delivery system, thereby forming a complex, and the resulting complex may be used as a gene delivery system.

In the present invention, the gene delivery system may include the above-described recombinant vector, and may be one of a virus-mediated system and a non-viral system, and preferably, a virus-mediated system.

### Ribozyme

Another aspect of the present invention provides a ribozyme expressed from a recombinant vector according to the present invention.

Descriptions of a recombinant vector or ribozyme according to the present invention are as described above.

### Pharmaceutical composition

Yet another aspect of the present invention provides a pharmaceutical composition for preventing or treating cancer, which includes a recombinant vector according to the present invention, a gene delivery system including the recombinant vector, or a ribozyme as an active ingredient.

Descriptions of the recombinant vector, gene delivery system, or ribozyme according to the present invention are as described above.

The term "cancer" used herein refers to a condition in which cells are abnormally over-proliferated due to problems occurring in the regulation of normal cell division, differentiation and death and infiltrate into surrounding tissue and organs, thereby forming lumps, and thus the original structures of the tissue or organ is destroyed or deformed.

The cancer according to the present invention is preferably liver cancer, glioblastoma, bile duct cancer, lung cancer, pancreatic cancer, melanoma, bone cancer, breast cancer, colon cancer, stomach cancer, prostate cancer, leukemia, uterine cancer, ovarian cancer, lymphoma, or brain cancer, more preferably, liver cancer, glioblastoma, or bile duct cancer, and most preferably, liver cancer.

In addition, according to the present invention, cancers preferably show the copy number (expression level) of miR-122 expressed therein less than 100 times the copy number of ribozymes expressed by the pharmaceutical composition in the cancer tissue.

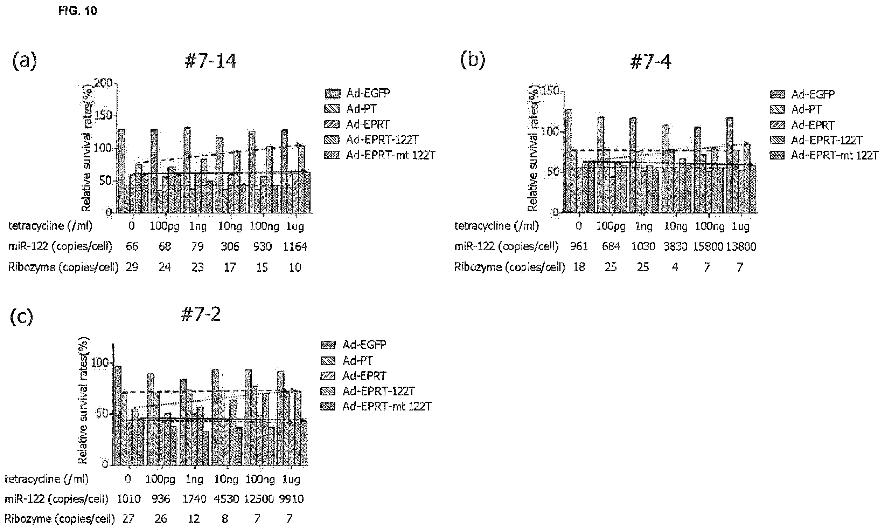

In one embodiment of the present invention, as a result of observing the expression level of an hTERT-targeting ribozyme by the vector including miR-122T sequence in comparison with the expression level of miR-122 in cells, as the ratio of the miR-122 expression to the ribozyme expression increased, the ribozyme expression decreased and consequently a cell death-inducing effect also decreased (FIGS. 4, 8 and 9). Accordingly, the injection amount of an adenovirus expressing a ribozyme may be determined by estimating the amount of the ribozyme that exhibits an anticancer effect according to the expression level of miR-122 in cancer tissue. Specifically, when the minimum copy number of miR-122 is approximately 100 times or higher the copy number of the ribozyme, the function (expression) of the ribozyme having a miR-122 target site is weakened, and when the copy number of miR-122 expressed in cancer tissue is less than 100 times the copy number of ribozymes expressed due to the pharmaceutical composition according to the present invention in the cancer tissue, high anticancer efficacy can be achieved (FIG. 10).

In addition, the cancer according to the present invention is preferably a cancer in which miR-122 is substantially not expressed in cancer tissue. The "cancer in which miR-122 is substantially not expressed in cancer tissue" refers to a cancer where miR-122 is expressed in a small copy number so that the miR-122 in cancer tissue does not exhibit a substantial effect on the function of a ribozyme having a miR-122 target site.

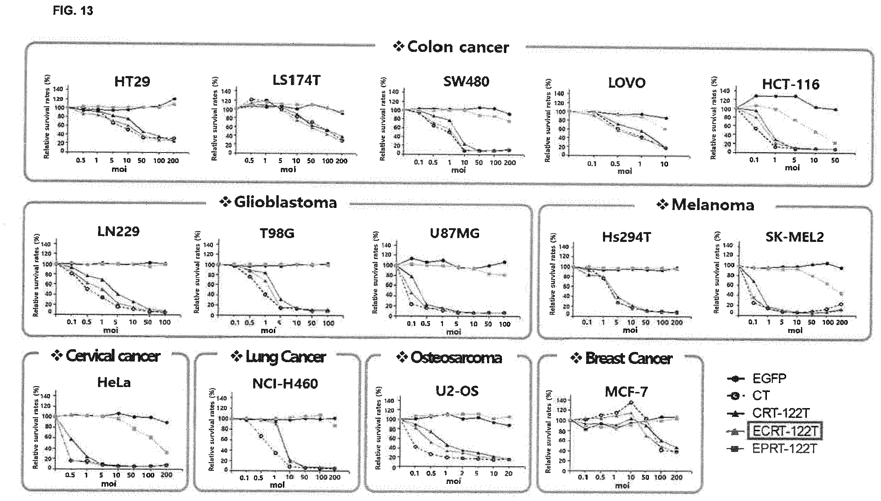

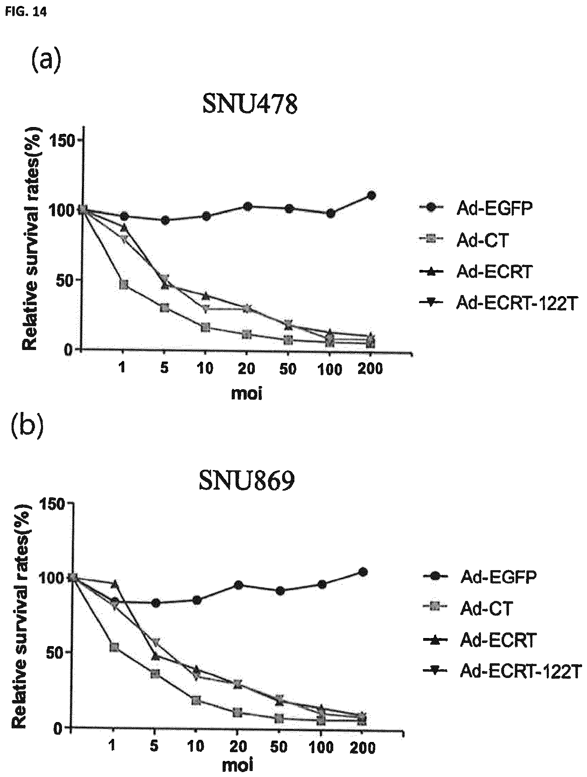

In one embodiment of the present invention, in colon cancer, glioblastoma, melanoma, cervical cancer, lung cancer, osteosarcoma, breast cancer and bile duct cancer cell lines in which miR-122 is not substantially expressed in the cancer tissue, the anticancer efficacy of a ribozyme according to the present invention was confirmed (FIGS. 13 and 14).

In addition, liver cancer according to the present invention is preferably caused by any one or more selected from the group consisting of the hepatitis B virus, the hepatitis C virus decreasing miR-122 expression in liver cancer tissue, alcohol, chronic hepatitis, liver cirrhosis, non-alcoholic fatty acid disease, aflatoxin, and family history.

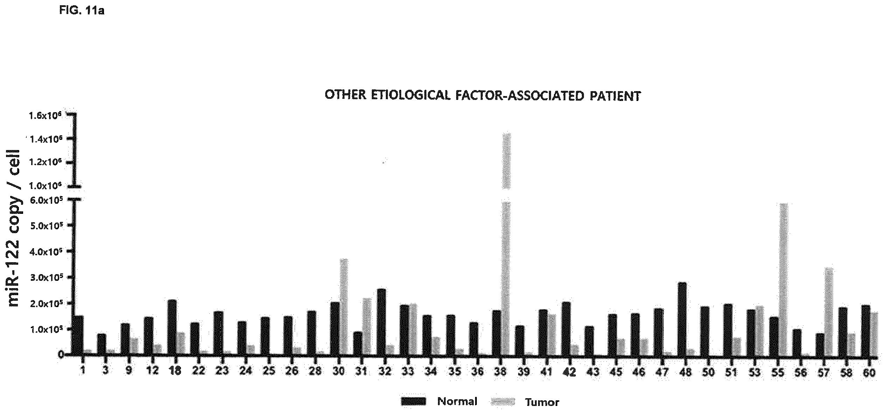

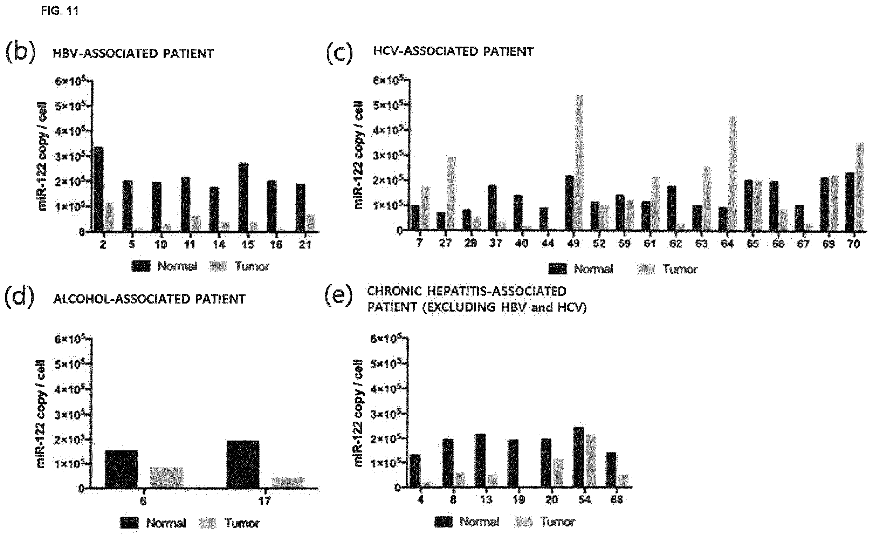

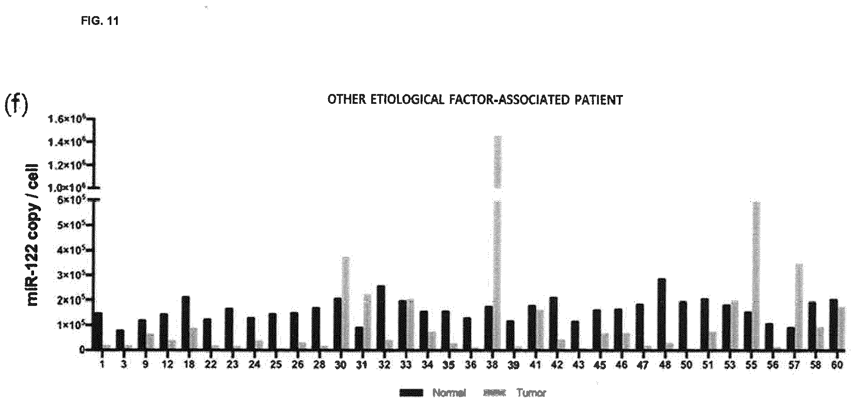

In one embodiment of the present invention, the expression levels of miR-122 in liver cancer caused by various etiological factors were analyzed. As a result, for some liver cancer caused by HCV and liver cancer caused by other factors, the miR-122 expression level in normal liver tissue was higher than the miR-122 expression level in liver cancer tissue. Therefore, it was confirmed that the activity of the ribozyme according to the present invention would be decreased in normal liver tissue due to high miR-122 expression level, while its high activity could be maintained in liver cancer tissue where miR-122 expression level is low (FIG. 11).

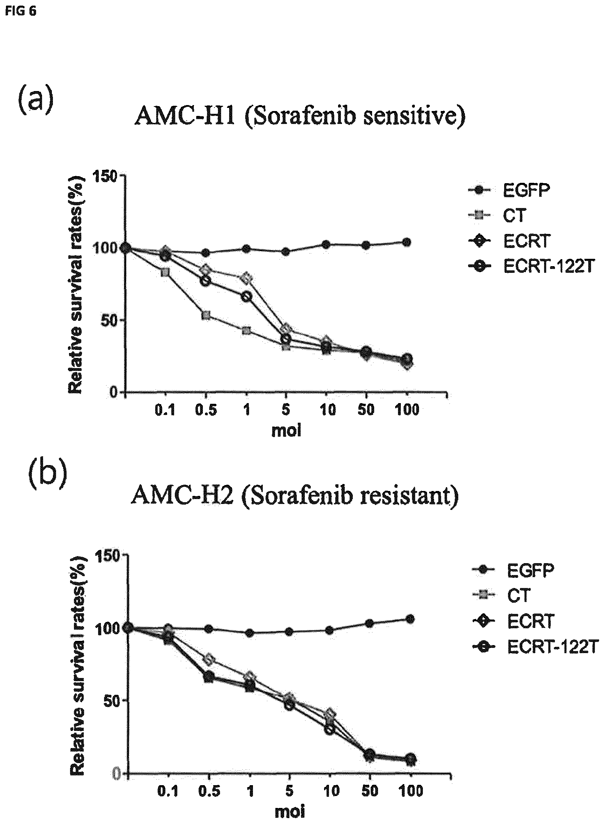

In addition, the liver cancer according to the present invention may be resistant to sorafenib. Sorafenib is a primary therapeutic agent used against advanced liver cancer, and in one embodiment of the present invention, since the ribozyme according to the present invention induces cell death in both a sorafenib-sensitizing cell line and a non-sorafenib-sensitizing cell line, it was confirmed that the ribozyme can also be applied to a sorafenib-resistant liver cancer patient group which cannot be treated by sorafenib (FIG. 6).

The term "prevention" used herein refers to all actions of inhibiting cancer or delaying the onset thereof as a result of administration of the composition including the recombinant vector according to the present invention.

The term "treatment" used herein refers to all actions involved in alleviating or beneficially changing symptoms of cancer by administration of the composition including the vector according to the present invention.

The pharmaceutical composition according to the present invention may further include a pharmaceutically acceptable carrier, excipient or diluent. Examples of a pharmaceutically acceptable carrier, excipient or diluent that can be used in the pharmaceutical composition according to the present invention may include lactose, dextrose, sucrose, sorbitol, mannitol, xylitol, erythritol, maltitol, starch, acacia gum, alginate, gelatin, calcium phosphate, calcium silicate, calcium carbonate, cellulose, methyl cellulose, polyvinylpyrrolidone, water, methylhydroxy benzoate, propylhydroxy benzoate, talc, magnesium stearate, and mineral oil.

The pharmaceutical composition of the present invention may be administered orally or parenterally, and preferably, parenterally according to a desired method.

According to one embodiment of the present invention, the pharmaceutical composition according to the present invention may be directly administered intravenously, intraarterily, intratumorally or subcutaneously, or in the form of an injection. The injection according to the present invention may be dispersed in a sterilized medium to be used without modification when being administered to a patient, and may be administered after being dispersed at a suitable concentration using distilled water for injections. In addition, when the pharmaceutical composition is prepared as an injection, it may be mixed with a buffer, a preservative, an analgesic, a solubilizer, an isotonic agent, a stabilizer or the like to be formed in a unit dose ampoule or multiple-dose form.

The dose of the pharmaceutical composition of the present invention may vary depending on the condition and body weight of a patient, the severity of a disease, a drug type, an administration route and time, and may be suitably selected by one of ordinary skill in the art. Meanwhile, the pharmaceutical composition according to the present invention may be used alone, or in combination with adjuvant therapy such as a surgical method.

Hereinafter, the present invention will be described in detail with reference to the following examples. However, examples according to the present invention may be modified into a variety of different forms, and it should not be construed that the scope of the present invention is limited to the following examples. The examples of the present invention are provided to more completely describe the present invention to those of ordinary skill in the art.

### Example 1: Construction of an hTERT-targeting trans-splicing ribozyme recombinant vector

To construct an hTERT-targeting trans-splicing ribozyme having miR-122T as a miR-122 target site and exhibiting a high expression level, a recombinant vector in which a conventional trans-splicing ribozyme expression vector is modified was designed.

Specifically, a CMV promoter was introduced into the conventional trans-splicing ribozyme expression vector which targets the +21 residue of hTERT mRNA, has an antisense sequence (SEQ ID NO: 8) of 326 nucleotides in length and has HSV-tk as a therapeutic gene. Transcription efficiency was enhanced by inserting a SV40 intron splicing donor/acceptor (SD/SA) sequence between the CMV promoter and the ribozyme-encoding gene. The protein expression efficiency of a therapeutic gene was improved by inserting WPRE, a posttranscriptional regulatory element of the Woodchuck hepatitis virus, at the 3'end region of the therapeutic gene HSV-tk, and regulated by miR-122 as a result of inserting three copies of miR-122T at the 3' end of the construct.

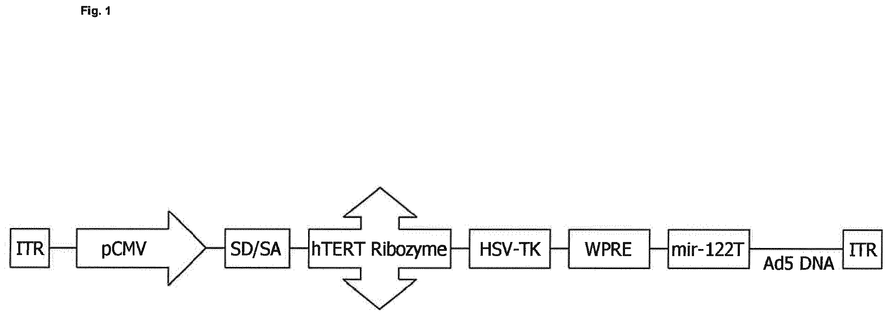

The entire vector structure of the trans-splicing ribozyme as designed is shown in FIG. 1, and was named ECRT-122T.

### Example 2: Construction of Hep3B stable cell line expressing miR-122

2×10⁵ Hep3B cells were seeded in a 35 mm culture dish, and cultured at 37 °C in a 5% CO₂ incubator. 1 µg of a Tet-repressor (TetR) miR-122 expressing vector and 100 µL of Opti-MEM were put into a 1.5 mL tube, and 5 µL of Lipofectamine 2000 and 100 µL of a serum-free medium were put into another 1.5 mL tube, and the tubes were mixed independently and then stored at room temperature for 5 minutes. Afterward, the contents in the two tubes were mixed together, and then stored at room temperature for 20 minutes to form a liposome-type composite. Twenty minutes later, the tube was centrifuged for 10 seconds, diffused on each cell for transfection, and 4 hours later, the medium was replaced with a fresh medium. After incubation for 24 hours at 37 °C in a 5% CO₂ incubator, the cells were washed with 1×PBS and treated with trypsin to detach the cells, and then the detached cells were transferred to a 100-mm culture dish and cultured. The medium was replaced with a medium containing 5 µg/mL of an antibiotic blasticidin once every 2 to 3 days. After cell clones were selected and grown, the expression of TetR by RT-PCR was confirmed.

### Experimental Example 1: Confirmation of anticancer efficacy of ECRT-122T (in vitro)

### 1-1. Comparison of anticancer efficacy of ECRT-122T according to miR-122 expression

It was confirmed that the anticancer efficacy of ECRT-122T constructed in Example 1 varies according to whether or not miR-122 is expressed in cancer cells.

A Hep3B cell line which expresses hTERT but does not express miR-122 and a Huh-7.5 liver cancer cell line which expresses both hTERT and miR-122 were prepared, and then 1x10⁴ cells derived from each cell line were seeded in a 96-well plate. The next day, according to the multiplicity of infection (MOI) to be treated to the cells, adenoviruses (type 5) were diluted by steps in a cell culture medium to reach a total volume of 100 µL, and then the resultant material was added to each well. Vectors used herein are as follows: ECRT-122T having miR-122T; ECRT not having miR-122T; and CRT consisting of a CMV promoter, an hTERT-targeting trans-splicing ribozyme and HSVtk. Twenty-four hours after the adenovirus treatment, ganciclovir (GCV) was diluted in a cell culture medium and mixed to reach a final volume of 200 µM, followed by treatment to each well. GCV was treated three times every 2 days, and 24 hours after the final GCV treatment, an MTS assay reagent was added, followed by measurement of absorbance at a wavelength of 450 nm in order to confirm living cells.

As a result, as shown in FIG. 2(a), in the Hep3B cell line not expressing miR-122, regardless of the presence or absence of a miR-122 target site (ECRT or ECRT-122T), the same degree of cell death was induced. The CRTs not having SD/SA, WPRE and miR-122T induced cell death, but was less effective than those of the ECRT and ECRT-122T, indicating that the efficiency of a ribozyme increases due to an element introduced into the ECRT and ECRT-122T.

However, as shown in FIG. 2(b), in the Huh-7.5 cell line expressing miR-122, an effect of inducing cell death by ECRT-122T was reduced, indicating that miR-122 acted on a miR-122 target site introduced into ECRT-122T by interrupting the ribozyme action. However, ECRT-122T still induced cell death at a similar degree to the level by the CRT-treated group, indicating that although being regulated by miR-122 due to miR-122T, ECRT-122T can exhibit an anticancer effect because of high expression of ribozyme, despite miR-122 expression in the cells.

### 1-2. Comparison of anticancer efficacy of ECRT-122T according to promoter

The anticancer effect of ECRT-122T constructed in Example 1 was compared in vectors with different configurations.

Specifically, Hep3B, SNU398 and SNU449 cell lines expressing hTERT but not expressing miR-122, and Huh-7 and Huh-7.5 liver cancer cell lines expressing both hTERT and miR-122 were prepared. Relative cell survival rates were compared after each of the cell lines were treated with ECRT-122T, ECRT, EPRT-122T in which a liver-specific promoter, that is, a PEPCK promoter, SD/SA, WPRE and miR-122T were introduced into CRT, or EPRT not having miR-122T. Cell proliferation analysis was performed by the same method as described in Experimental Example 1-1.

As a result, as shown in FIG. 3, in the cell line not expressing miR-122, regardless of the presence or absence of a miR-122 target site (ECRT or ECRT-122T), the same degree of cell death was induced. ECRT-122T induced a higher rate of cell death even in a sample treated with a viral MOI lower than EPRT-122T, indicating that high anticancer efficacy can be exhibited even in a sample treated with a small amount of a virus as a result of more highly increasing the expression of a ribozyme by a CMV promoter compared to a tissue-specific promoter, that is, PEPCK. In addition, ECRT-122T exhibited the same anticancer efficacy as that of the miR-122T-free ECRT, indicating that no decrease in ribozyme activity is caused by the introduction of miR-122T.

Meanwhile, as shown in FIG. 4, cell death caused by EPRT-122T was not observed in a cell line expressing miR-122, indicating that ribozyme expression is reduced by miR-122 acting on miR-122T. However, ECRT-122T induced cell death despite the expression of miR-122, indicating that the CMV promoter can increase ribozyme expression more powerfully than PEPCK. However, unlike a cell line not expressing miR-122, a degree of cell death caused by ECRT-122T was lower than that related to ECRT, indicating that the ribozyme expression was lowered by regulating miR-122T of ECRT-122T by miR-122.

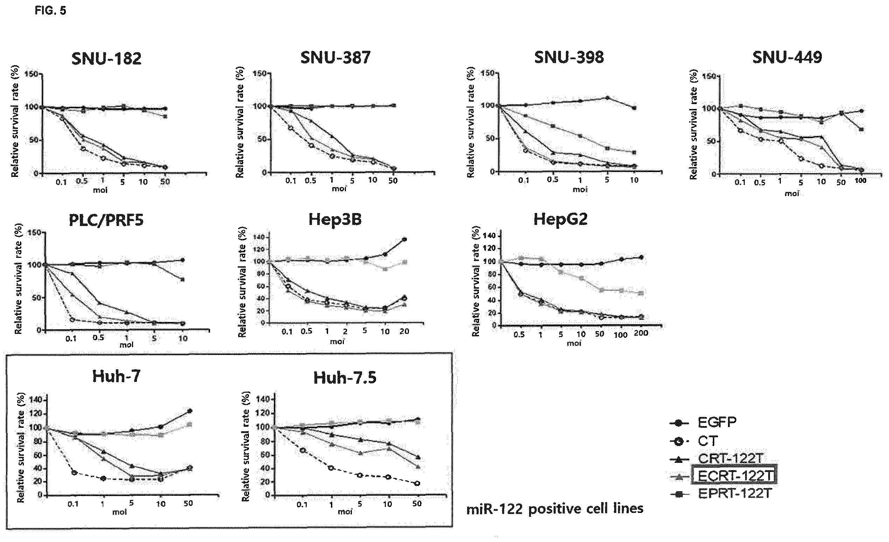

In addition, the anticancer effect of CRT-122T, ECRT-122T and EPRT-122T was compared in various liver cancer cell lines. As a result, as shown in FIG. 5, cell death increased by increasing the MOI of ECRT-122T in almost all liver cancer cell lines, and the anticancer effect of ECRT-122T was higher than that of the Comparative Groups (CRT-122T and EPRT-122T).

From the results of Experimental Examples, it can be seen that since anticancer efficacy is increased by increasing ribozyme expression as a result of introducing a CMV promoter, SD/SA and WPRE into a ribozyme-expression vector, and ribozyme activity caused by miR-122 is regulated by the introduction of miR-122T, ECRT-122T is considered as an excellent anticancer agent with enhanced efficacy as well as safety in regards to the treatment of liver cancer.

### 1-3. Application of ECRT-122T to sorafenib-resistant liver cancer

The anticancer efficacy of ECRT-122T against liver cancer sensitized to sorafenib, which was used as a primary therapeutic agent against advanced liver cancer, was confirmed.

Specifically, a cell line derived from a liver cancer patient having sorafenib sensitivity or a liver cancer patient without sorafenib sensitivity (resistance) was prepared, and following treatment with ECRT-122T or ECRT, cell survival rates were compared. Cell proliferation analysis was performed by the same method as that described in Experimental Example 1-1.

As a result, as shown in FIG. 6, cell death was observed in all cell lines with and without sorafenib sensitivity. It can be seen that ECRT-122T can also be applied to a sorafenib-resistant liver cancer patient group which shows resistance to sorafenib treatment.

### Experimental Example 2: Confirmation of anticancer efficacy of ECRT-122T (in vivo)

Anticancer efficacy and hepatotoxicity caused by ECRT-122T in animal models towards liver cancer were confirmed.

Anticancer efficacy was confirmed as a result of inducing orthotopic multifocal liver cancer by injecting a Hep3B cell line not expressing miR-122 into the spleen of a mouse model with metastasized liver cancer in the spleen, and systemically administering the adenovirus expressing ECRT or ECRT-122T by tail-vein injection and injecting GCV

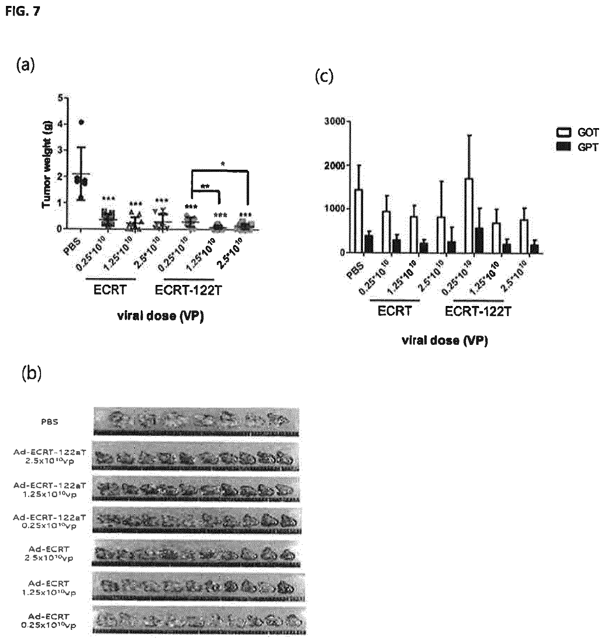

Consequently, as shown in FIGS. 7(a) and 7(b), most types of cancer cells were killed in an ECRT-122T-injected experimental group, compared with a control group (injected with PBS). Here, ECRT-122T exhibited more excellent anticancer efficacy than ECRT not having miR-122T in the experimental groups injected with the same dose of viruses. From the result, it can be seen that since ribozyme expression is inhibited in normal liver tissue due to the introduction of miR-122T, safety can be ensured within a normal liver, and high anticancer efficacy can be exhibited in cancer tissue. In addition, as shown in FIG. 7(c), compared with the control group (injected with PBS), an ECRT-122T-injected experimental group showed a similar hepatotoxicity level, indicating that *in vivo* delivery of ECRT-122T using an adenovirus is appropriate.

### Experimental Example 3: Correlation between miR-122 expression level and ribozyme expression level

To analyze the action of a ribozyme according to the expression level of miR-122, the correlation between the miR-122 expression level and the ribozyme expression level was analyzed.

Huh-7 and Huh-7.5 liver cancer cell lines expressing both hTERT and miR-122 were prepared and treated with adenoviruses expressing ECRT, ECRT-122T, EPRT or EPRT-122T, followed by extracting 5 µg of total RNA with a TRI Reagent.

cDNA was synthesized by reverse transcription performed on the extracted total RNA with a random primer (5'-NNNNNN-3'), and a 10x PCR buffer, 10 mM of dNTP, 10 pmole of each primer, 2.5 units of a Taq polymerase and 1 µL of 10x SYBR Green were added to 2 µL of the synthesized cDNA and diluted with dH₂O to reach a final volume of 70 µL. 20 uL of the resulting mixture was dispensed in each well, and subjected to 40 cycles of real-time PCR under conditions of 95 °C for 30 seconds, 58 °C for 30 seconds and 72 °C for 30 seconds to confirm the expression level of a ribozyme. Primer sequences used in the PCR for a ribozyme are shown in Table 1 below.

**[Table 1]**

| **Primer sequence (5'-3')** | **Sequence (5'-3')** |
|---|---|
| Ribozyme_forward primer | TTCCGGAGGACAGACACATCGA (SEQ ID NO: 10) |
| Ribozyme_reverse primer | GCAGATACCGCACCGTATTGGC (SEQ ID NO: 11) |

Subsequently, cDNA was synthesized from the extracted total RNA using a mature miR-122 probe (ABI), and 3.5 µL of 20x TaqMan small RNA assay and 35 µL of 2x TaqMan Universal PCR Master Mix II (ABI) were added to 2 µL of the synthesized cDNA and then diluted with dH₂O to reach a final volume of 70 µL. 20 uL of the resulting mixture was dispensed in each well, and subjected to 40 cycles of real-time PCR under conditions of 95 °C for 15 seconds and 60 °C for 60 seconds to confirm the expression level of miR-122. PCR of miR-122 was performed using a TaqMan probe provided by ABI (Assay ID: 002245).

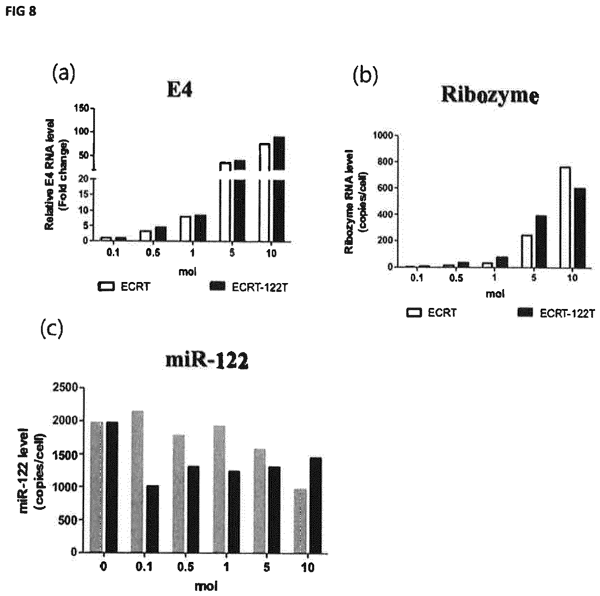

As a result, as shown in FIG. 8(c), it was confirmed that approximately 1000 to 2000 copies of miR-122 were expressed in the Huh-7 cell line, and as shown in FIG. 8(b), it can be seen that the expression level of a ribozyme increases according to an increase of MOI in each of the ECRT-122T and ECRT-treated groups. In addition, the ribozyme is expressed more greatly at an infection concentration of 5 MOI or less in the ECRT-122T-treated group, compared with the ECRT-treated group, but when 10 MOI was treated, the ribozyme expression level was lowered in the ECRT-122T-treated group, compared with the ECRT-treated group. From the result, it can be seen that Huh-7-expressing miR-122 acted on miR-122T to reduce ribozyme expression.

Meanwhile, when ECRT-122T was treated at 10 MOI, a ratio of the copy numbers of the ribozyme and miR-122 was approximately 1:2.5 (600 copies: 1500 copies). In FIG. 4(a) confirming a cell death rate according to MOI, it can be seen that the induction of cell death by ECRT-122T even at 10 MOI was caused by the ribozyme expression due to miR-122 not being sufficiently inhibited as a result of a low ratio of miR-122 to the ribozyme.

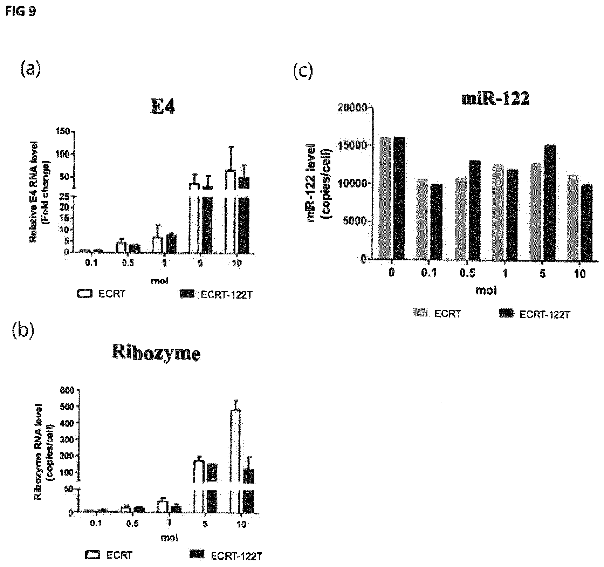

Meanwhile, as shown in FIG. 9(c), approximately 10000 to 15000 copies of miR-122 were expressed in a Huh-7.5 cell line, which was a higher level than that of Huh-7, and it can be seen that a degree of inducing cell death by a ribozyme was lower than that of Huh-7. In addition, as shown in FIG. 9(b), as opposed to Huh-7, under the same infection condition, the ribozyme expression level in the ECRT-treated group was the same or higher than that in the ECRT-122T-treated group, and when 10 MOI was treated, the copy number of the expressed ribozyme was lower (approximately 120 copies) than that in Huh-7 (approximately 600 copies). From the above-mentioned results and the result of FIG. 4(b) showing a cell death rate according to MOI, it can be seen that as the ratio of a ribozyme and miR-122 increased, the ribozyme expression decreased, and thus an effect of inducing cell death was reduced.

### Experimental Example 4: Action of ribozyme according to expression level of miR-122

To further analyze the interaction between the expression level of miR-122 and a ribozyme, the action of the ribozyme according to the expression level of miR-122 was analyzed.

After a stable cell line (Hep3B) having the miR-122 tetracycline-on system, which was constructed in Example 2, was treated with an adenovirus containing EPRT, EPRT-122T, or EPRT-mt 122T vector having a mutant miR-122 target site in which the sequence was randomly changed to prevent miR-122 binding, cell survival rates were compared. There was in increase in miR-122 expression in the cell line constructed in Example 2 based on a tetracycline treatment concentration, and an experiment was performed using a stable cell line of three clones with different expression levels. Cell proliferation analysis was performed by the same method as described in Experimental Example 1-1.

As a result, as shown in FIG. 10, in the EPRT-122T-treated group, as the tetracycline treatment concentration increased, the miR-122 copy number increased, and thus cell death caused by EPRT-122T was reduced. However, in the case of the EPRT or EPRT-mt 122T treated group, or a positive control PT group, the cell death-inducing effect was maintained regardless of the miR-122 expression level. In addition, as the tetracycline treatment concentration increased, the miR-122 copy number increased, and thus the ribozyme copy number decreased. The above-mentioned effect was the same with respect to all of the three independent clones used in the experiment.

The correlation between the miR-122 copy number and the ribozyme copy number, confirmed in the experiment, was quantified. As a result, as shown in Table 2 below, while the ratio varies according to cell line, when the minimum copy number of miR-122 is approximately 100 times or higher compared to the ribozyme copy number, the activity of a ribozyme having an miR-122 target site was significantly degraded. From the above-mentioned result, it can be seen that since the expression of a trans-splicing ribozyme having an miR-122 target site can be regulated by miR-122, an injection amount of adenoviruses expressing a ribozyme can be determined by estimating an amount of the ribozymes needed to exhibit an anticancer effect according to the expression level of miR-122.

**[Table 2]**

| Cell | miR-122 copies/cell | | Ribozyme copies/cell | | Cell death rate (%) | | miR-122/ ribozyme (fold) | |
|---|---|---|---|---|---|---|---|---|
| | Tetracycline | | Tetracycline | | Tetracycline | | Tetracycline | |
| | - | + | - | + | - | + | - | + |
| Hep3B miR-122 stable cell line #7-2 | 1,010 | 9,910 | 27 | 7 | 46 | 29 | 37 | 1,416 |
| Hep3B miR-122 stable cell line #7-4 | 961 | 13,800 | 18 | 7 | 40 | 14 | 53 | 1,971 |
| Hep3B miR-122 stable cell line #7-14 | 66 | 1164 | 29 | 10 | 25 | 0 | 2.6 | 116 |
| Huh-7 | 11,600 | | 29 | | 0 | | 400 | |
| Huh-7.5 | 66,300 | | 111 | | 0 | | 597 | |

### Experimental Example 5: Analysis of miR-122 expression in liver cancer patient tissue

The miR-122 expressions in normal liver tissue and liver cancer tissue of a liver cancer patient were analyzed by real-time PCR.

Specifically, miR-122 expression was analyzed by receiving samples of tissue from 70 liver cancer patients in Korea. Each of a normal liver tissue and a liver cancer tissue of a liver cancer patient was immersed in 1 mL of a TRIzol solution, homogenized, and left for 5 minutes at room temperature. Afterward, 0.2 mL of chloroform was added, left alone for 3 minutes at room temperature, and centrifuged for 30 minutes at 4 °C and 12,000 rpm to only separate a supernatant. 0.5 mL of isopropanol was added to the separated supernatant and reacted at -20 °C, and the resulting mixture was centrifuged again for 20 minutes at 4 °C and 14,000 rpm to obtain an RNA pellet, which was then dissolved in 100 µL of RNase-free water. cDNA was synthesized by reverse transcription from 50 ng of extracted RNA using a TaqMan miRNA Reverse Transcription kit, and 1 µL of the synthesized cDNA was mixed with a TaqMan 2X Universal PCR Master Mix and subjected to PCR using an ABI StepOne Plus instrument. PCR was performed using a TaqMan probe provided by ABI (Assay ID: 002245).

As a result, as shown in FIG. 11(a), the miR-122 expression level in the liver cancer tissue was lower than that in the normal liver tissue in 48 (68.6%) of the 70 patients. According to etiological analysis, as shown in FIGS. 11(b) and 11(d) to 11(f), miR-122 was expressed at a low level in liver cancer tissue, compared with normal liver tissue, in 8 of 8 HBV-associated patients, 2 of 2 alcohol-associated patients, 6 of 7 chronic hepatitis-associated patients, and 26 of 36 liver cancer patients with a cause based on other etiological factors. However, as shown in FIG. 11(c), miR-122 was expressed at a low level in liver cancer tissue in only 6 of 18 HCV-associated patients. From this result, it can be seen that in patients with advanced liver cancer caused by HCV, as opposed to those caused by other etiological factors, miR-122 expression increased or was maintained.

In addition, according to statistical analysis, as shown in Table 3 below, in the case of HCV-associated patients, a phenomenon in which the miR-122 expression is highly observed in liver cancer tissue, compared with normal liver tissue, was shown to be statistically significant, and in the case of another hepatitis virus HBV, a phenomenon in which miR-122 expression decreases in all types of liver cancer tissue is shown to be statistically significant. From this result, it can be seen that the maintenance or increase in miR-122 expression in liver cancer tissue of a HCV-associated patient was statistically significant, and is a HCV-specific phenomenon, not a general phenomenon caused by a hepatitis virus.

**[Table 3]**

| **Etiological factor** | **Patient Number (n=70)** | **miR-122 level normal > cancer** | **miR-122 level normal <= cancer** | ***P* value** |
|---|---|---|---|---|
| **Group 1** | | | | 0.0021 |
| HBV | 8 (11.4%) | 8 (100%) | 0 (0%) | |
| HCV | 18 (25.7%) | 6 (33.3%) | 12 (66.7%) | |
| Alcohol | 2 (2.9%) | 2 (100%) | 0(0%) | |
| Chronic hepatitis | 7 (10%) | 6 (85.7%) | 1 (14.3%) | |
| Other | 35 (50%) | 26 (74.3%) | 9 (25.7%) | |
| **Group 2** | | | | 0.0498 |
| Non-HBV | 62 (88.6%) | 40 (64.5%) | 22 (35.5%) | |
| HBV | 8(11.4%) | 8 (100%) | 0(0%) | |
| **Group 3** | | | | 0.0007 |
| Non-HCV | 52 (74.3%) | 42 (80.8%) | 10 (19.2%) | |
| HCV | 18 (25.7%) | 6 (33.3%) | 12 (66.7%) | |

From this result, it can be seen that when ECRT-122T was applied to patients with various types of liver cancer, excluding those caused by HCV with an increase in miR-122 expression in liver cancer tissue compared to normal liver tissue, because the ribozyme does not act on the normal liver tissue due to miR-122 but acts on liver cancer tissue having decreased miR-122 expression, a cancer-specific anticancer action may be exhibited.

In addition, the mRNA and protein levels of hTERT and the miR-122 level of a liver cancer cell line derived from liver cancer cells of a patient were analyzed. A liver cancer cell line was incubated to isolate RNA and protein by a method known in the art. The RNA was used for qRT-PCT, and the protein was subjected to electrophoresis and western blotting.

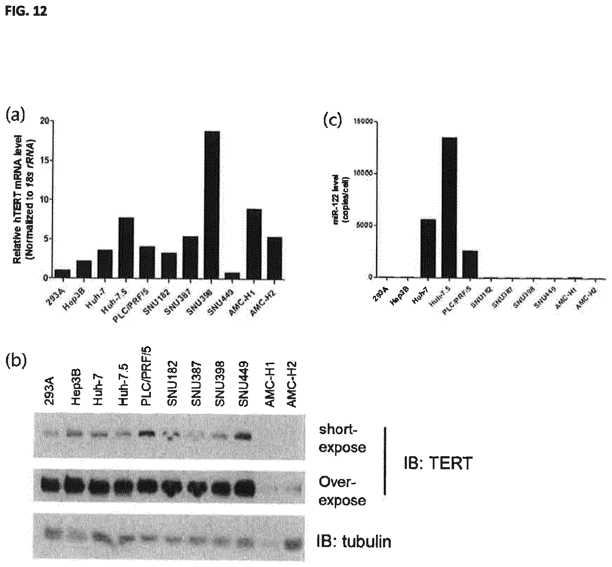

As a result, as shown in FIGS. 12(a) and 12 (b), it can be confirmed that mRNA of hTERT, which is a target molecule of ECRT-122T, was expressed in most of the liver cancer cell lines, and an hTERT protein level showed a similar result. In addition, similar to results showing reduced expression in liver cancer tissue, it was confirmed that miR-122 was hardly expressed in each of the liver cancer cells line (FIG. 12(c)).

### Experimental Example 6: Application to tumors not expressing miR-122, other than liver cancer

### 6-1. Cell experiment (in vitro)

The anticancer efficacy of ECRT-122T was confirmed in a cancer cell line of tissue not substantially expressing miR-122.

Specifically, a glioblastoma cell line, a colon cancer cell line, a melanoma cell line, a cervical cancer cell line, a lung cancer cell line, an osteosarcoma cell line, a breast cancer cell line and a bile duct cancer cell line, which express hTERT, were treated with an ECRT-122T-expressing adenoviruses, and cell survival rates were compared. Cell proliferation analysis was performed by the same method as that described in Experimental Example 1-1.

As a result, as shown in FIG. 13, as the MOI increased in almost all cancer cell lines, cell death increased, and the anticancer effect of ECRT-122T was superior to that in the Comparative Groups (CRT-122T and EPRT-122T). Specifically, EPRT-122T including a liver tissue-specific promoter often failed to induce cell death, and CRT-122T induced cell death but had lower efficiency than ECRT-122T.

In glioblastoma cell lines T98G and U87MG, approximately 90% cell death was shown at approximately 1 MOI or less, and in a LN229 cell line, approximately 90% cell death was shown at 5 MOI or more. In addition, in a cervical cancer cell line HeLa, 90% or more cell death was shown at 0.5 MOI or less, and in a melanoma cell line SK-MEL2, approximately 90% cell death was confirmed at approximately 1 MOI or less.

In addition, as shown in FIG. 14, in bile duct cancer cell lines SNU478 and SNU869, as the MOI increased, cell death increased.

### 6-2. Animal experiment (in vivo)

1x10⁷ (100 µL) cells of a glioblastoma cell line LN229 or 5x10⁶ (100 µL) cells of a U87MG cell line were subcutaneously injected into 6-week-old male Balb/c-nunu mice to induce tumorigenesis. When tumors had grown to a certain size, ECRT-122T adenoviruses were injected at a dose of 1.0x10⁹ VP (100 µL) three times every 2 days. Twenty-four hours after the first virus injection, GCV was administered at a dose of 50 mg/kg twice a day for 10 days (a total of 20 times).

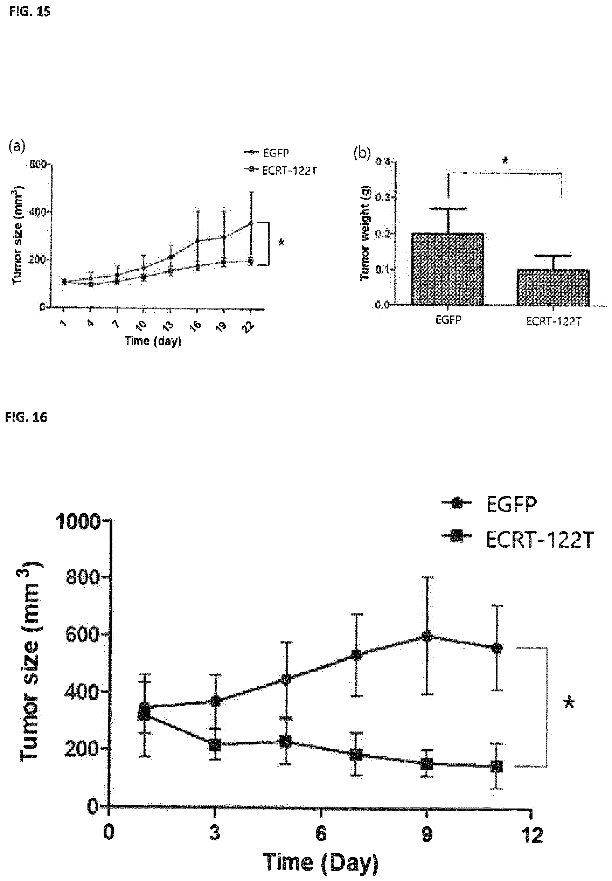

As a result of the experiment, as shown in FIG. 15, it can be seen that a tumor size gradually increased in a control group (injected with Ad-EGFP), but the tumor hardly grew in an ECRT-122T-injected experimental group, and a tumor weight was lower in the ECRT-122T-injected experimental group in all cases. In addition, as shown in FIG. 16, the tumor growth inhibitory effect of ECRT-122T was confirmed even in the experiment using the U87MG cell line.

From the result of Experimental Example 6, it was confirmed that a ribozyme expressed from ECRT-122T can be effectively applied to various carcinomas other than liver cancer that do not express miR-122. In addition, when ECRT-122T was systemically or locally administered as an anti-cancer agent against other types of cancer other than liver cancer, it can be introduced into a normal liver, and at that time, due to the action of miR-122T, the induction of toxicity in a normal liver may be inhibited.

### Experimental Example 7: Confirmation of in vivo distribution of ECRT-122T

### 7-1. Intravenous injection

ECRT-122T adenoviruses were injected into normal ICR mice at a dose of 2.5×10¹⁰ VP by intravenous injection, and then, 8, 11 and 15 days later, each major organ was isolated to extract DNA. The extracted DNA was used as a template, and subjected to PCR using primer sets for detecting a ribozyme to confirm ECRT-122T distribution in major tissue.

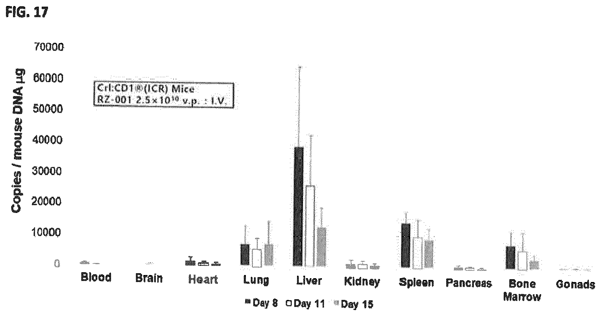

As a result, as shown in FIG. 17, it can be confirmed that almost all injected adenoviruses were distributed in the liver, indicating that adenoviruses are appropriate for use in the delivery of ECRT-122T to the targeted liver cancer. In addition, the injected ECRT-122T had completely disappeared from the blood by day 11 after the systemic introduction, and beginning at 8 days after the systemic injection and within two weeks of the injection, 70% of viral DNA had disappeared.

### 7-2. Hepatic artery injection

ECRT-122T adenoviruses were injected into rats at a dose of 2.5×10¹¹ VP by hepatic artery injection, and ECRT-122T distribution in major tissue was confirmed by the same method as that described in 7-1.

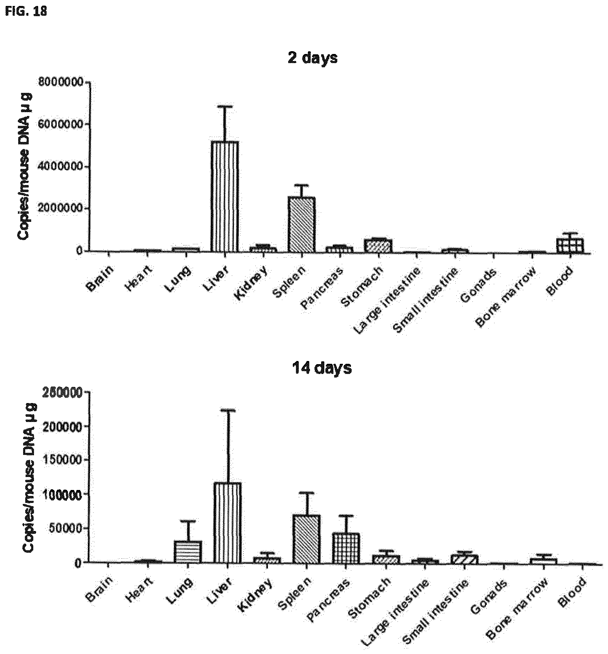

As a result, as shown in FIG. 18, on the second day after the injection, the largest amount of ECRT-122T DNA was detected in the liver, a level which was maintained up to 14 days after injection. 98% of the viral DNA had disappeared from the liver by day 14 after the injection, and almost all of the viral DNA had also disappeared from other tissues.

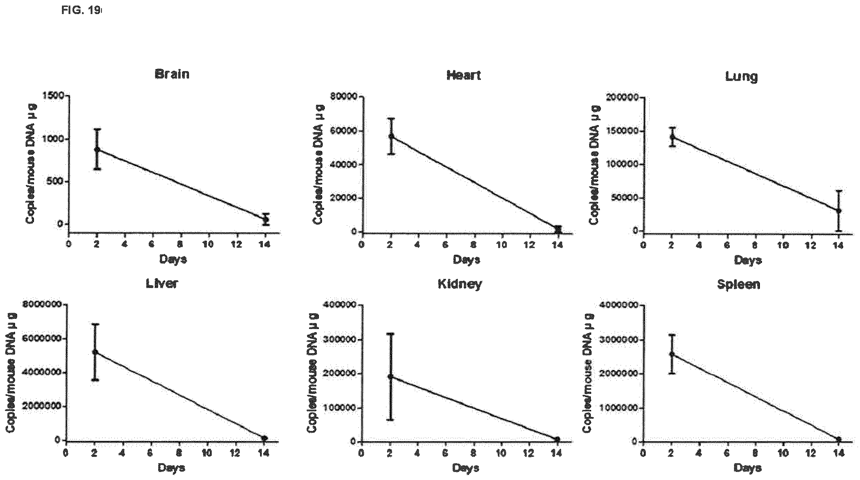

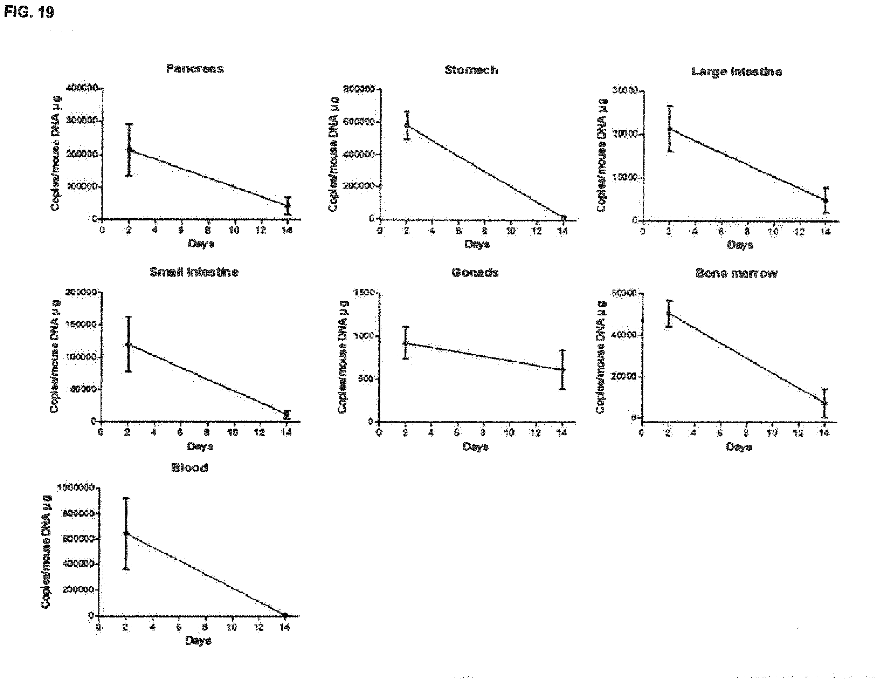

In addition, genomic DNA (gDNA) was isolated from each organ of a rat to quantify HSVtk DNA. As a result, as shown in FIG. 19, it can be seen that HSVtk DNA had disappeared from almost all organs by day 14 after the injection.

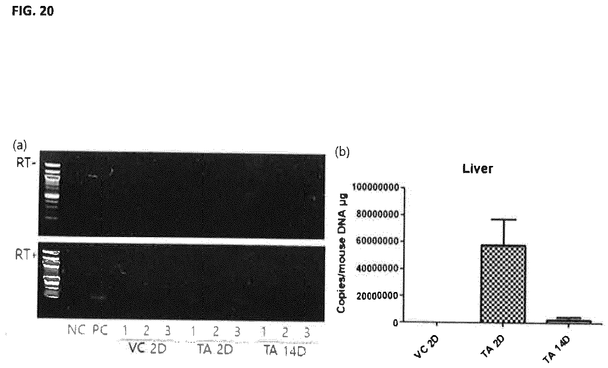

Total RNA was extracted from the liver of a rat, and a degree of ribozyme expression was identified by qRT-PCR. As a result, as shown in FIG. 20(a), it can be seen that a ribozyme was expressed starting at two days after ECRT-122T injection, and it can be confirmed that only approximately 3.85% of ribozymes were expressed on day 14 after the injection, compared with day 2 after the injection. As also confirmed from the genomic DNA, it can be seen that as shown in FIG. 20(b), compared with day 2 after injection, almost all gDNA of the adenovirus had disappeared, leaving approximately 2.23%, in day 14.

From the above-mentioned result, it can be seen that the *in vivo* distributions of the ECRT-122T adenovirus in the body show a similar pattern regardless of intravenous injection or intraarterial injection, and by day 14 after the injection, almost all of the viral DNA had disappeared.

### Experimental Example 8: Confirmation of ECRT-122T toxicity according to the performance or nonperformance of GCV treatment

### 8-1. Without GCV treatment

The ECRT-122T adenovirus was injected into normal ICR mice once, and on days 15 and 29 after the injection, AST and ALT levels were measured.

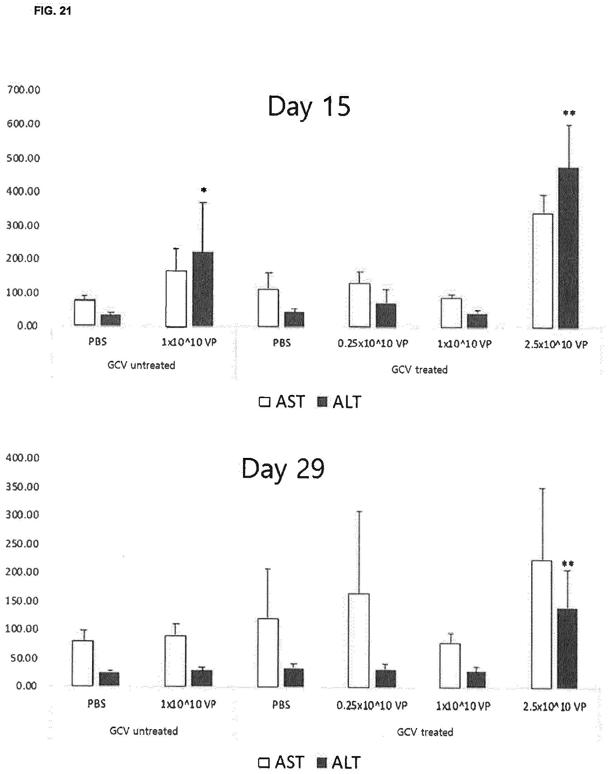

As a result, as shown in FIG. 21, it can be seen that weak toxicity was observed in a 2.5×10¹⁰ VP-injected experimental group, and in other experimental groups injected with lower doses, AST and ALT levels were observed to be at a level similar to those of a PBS-injected group, indicating that there was almost no toxicity.

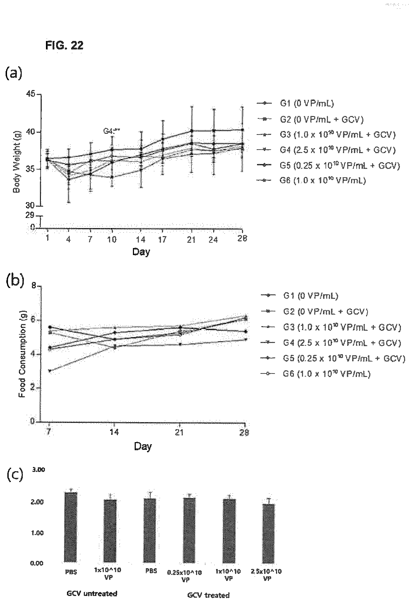

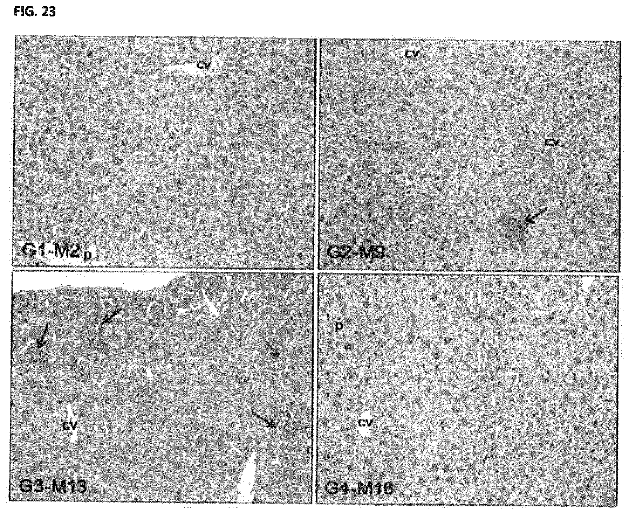

In addition, as shown in FIG. 22, it can be confirmed that there were no abnormal findings in terms of body weight, feed consumption, and liver weight of mice during the experiment. From the result of the histopathological examination performed twenty-nine days after the adenovirus was injected intravenously at one instance, as shown in FIG. 23, in the 2.5×10¹⁰ VP-injected experimental group (G3), hepatocellular necrosis (arrow) and inflammation were observed, indicating that minor liver damage occurred. In the 1.0×10¹⁰ VP-injected experimental group (G2), it was confirmed that local inflammatory cell infiltration (arrow) had occurred, but liver damage did not occur. In FIG. 23, cv indicates a central vein, and p indicates a portal area.

The histopathological examination results are summarized in Table 4 below.

**[Table 4]**

| **Histopathology /Groups** | **G1** | **G2** | **G3** | **G4** |
|---|---|---|---|---|
| | **0.5x10¹⁰** | **1.0x10¹⁰** | **2.5x10¹⁰** | **Vehicle (PBS)** |
| **No. examined** | **4** | **5** | **5** | **5** |
| No specific lesion | 4 (100) | 4 (80) | 0 (0.00) | 5 (100) |
| Inflammatory foci with hepatocyte necrosis | 0 (0.00) | 1 (20.0) | 4 (80.0) | 0 (0.00) |
| *Grades: Minimal* | 0 | 1 | 3 | 0 |
| *Mild* | 0 | 0 | 1 | 0 |
| Cell infiltration, mononuclear cells, periductal, focal | 0 (0.00) | 0 (0.00) | 2 (40.0) | 0 (0.00) |
| *Grades: Minimal* | 0 | 0 | 2 | 0 |

### 8-2. With GCV treatment

The ECRT-122T adenovirus was injected into normal ICR mice, and GCV was administered twice a day for 10 days, followed by measuring AST and ALT levels.

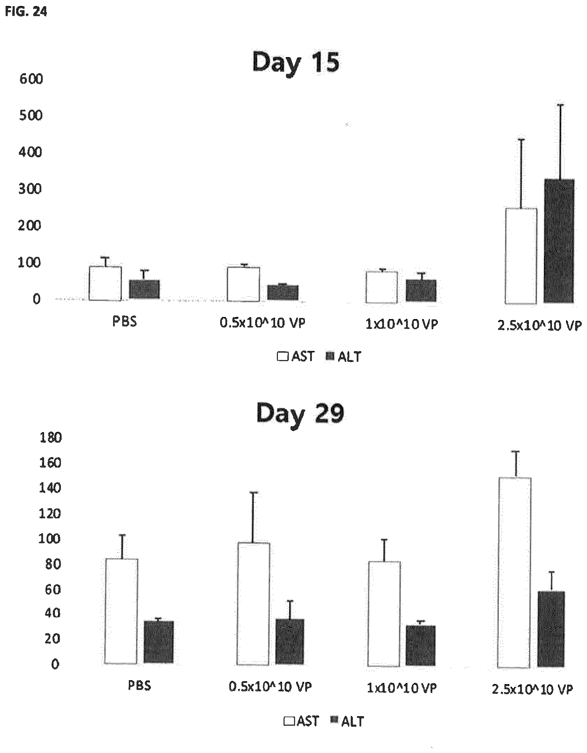

As a result, as shown in FIG. 24, in experimental groups injected at different doses, with the exception of the 2.5×10¹⁰ VP-injected experimental group, AST and ALT levels were observed to be at a level similar to those of a PBS-injected group, indicating that there was almost no toxicity.

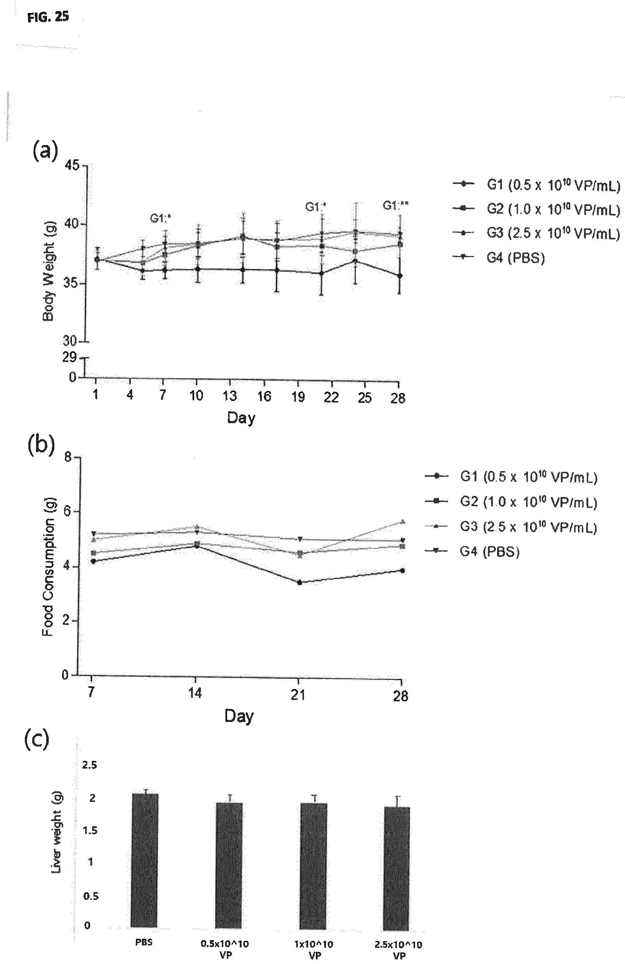

In addition, as shown in FIGS. 25(a) and 25(b), it can be confirmed that there were no significant changes in body weight, feed consumption and liver weight of mice during the experiment, and as shown in FIG. 25(c), there was no abnormal finding related to liver weight.

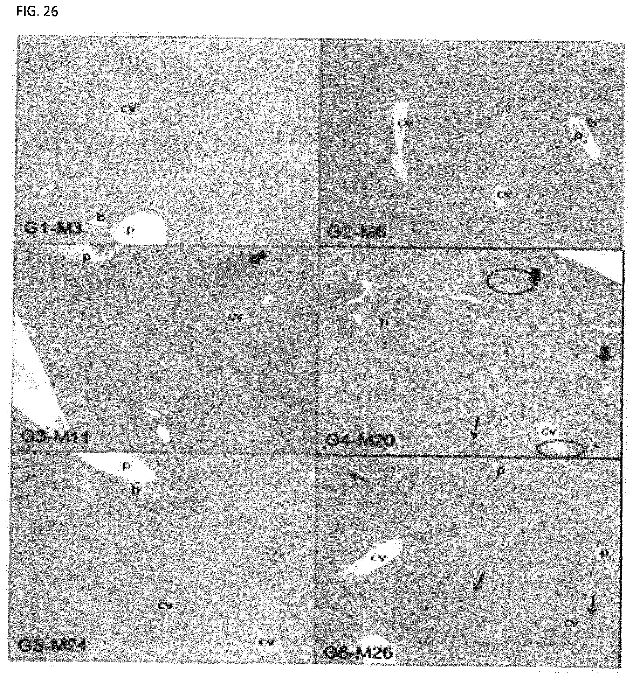

According to the histopathological analysis of the liver, as shown in FIG. 26, it can be confirmed that no abnormal findings were observed in the PBS-injected group (G1), the PBS+GCV-administered group (G2) and the 0.25×10¹⁰ VP+GCV-administered group (G5). However, in the 1.0×10¹⁰ VP+GCV-administered group (G3), a local microabscess (thick arrow) formed by neutrophil infiltration was observed, but liver damage did not occur. In the 2.5×10¹⁰ VP+GCV-administered group (G4), enlarged hepatocytes having a large nucleus, necrotic hepatocytes (thick arrow), multiple inflammatory cell infiltration (circle), increased hepatotic mitosis (thick arrow) and lymphatic cell infiltration into the proximity of the bile ductile (b) were observed, indicating the occurrence of liver damage. In G6 not administered with GCV (1.0×10¹⁰ VP-injected group), the number of mitotic hepatocytes (arrow) slightly increased.

The histopathological examination results are shown in Table 5 below.

**[Table 5]**

| **Histopathology /Groups** | **G1** | **G2** | **G3** | **G4** | **G5** | **G6** |
|---|---|---|---|---|---|---|
| | **Vehicle (PBS)** | **PBS+GCV** | **1.0x10¹⁰ +GCV** | **2.5x10¹⁰ +GCV** | **0.25x10¹⁰ +GCV** | **1.0x10¹⁰** |
| **No. examined** | **5** | **5** | **5** | **5** | **5** | **5** |
| No specific lesion | 5 (100) | 4 (80.0) | 3 (60.0) | 0 (0.00) | 5 (100) | 3 (60.0) |
| Cell infiltration, mononuclear or mixed cells, multifocal | 0 (0.00) | 1 (20.0) | 1 (20.0) | 4 (80.0) | 0 (0.00) | 0 (0.00) |
| *Grades: Minimal* | 0 | 1 | 1 | 0 | 0 | 0 |
| *Mild* | 0 | 0 | 0 | 4 | 0 | 0 |
| Microabcess, focal | 0 (0.00) | 0 (0.00) | 1 (20.0) | 0 (0.00) | 0 (0.00) | 0 (0.00) |
| *Grades: Minimal* | 0 | 0 | 1 | 0 | 0 | 0 |
| Hepatocytomegaly, diffuse | 0 (0.00) | 0 (0.00) | 0 (0.00) | 5 (100) | 0 (0.00) | 0 (0.00) |
| *Grades: Minimal* | 0 | 0 | 0 | 1 | 0 | 0 |
| *Mild* | 0 | 0 | 0 | 2 | 0 | 0 |
| *Moderate* | 0 | 0 | 0 | 2 | 0 | 0 |
| Necrotic hepatocytes | 0 (0.00) | 0 (0.00) | 0 (0.00) | 3 (60.0) | 0 (0.00) | 0 (0.00) |
| *Grades: Minimal* | 0 | 0 | 0 | 2 | 0 | 0 |
| *Mild* | 0 | 0 | 0 | 1 | 0 | 0 |
| Hepatocytic mitosis, | 0 (0.00) | 0 (0.00) | 0 (0.00) | 4 (80.0) | 0 (0.00) | 1 (20.0) |
| increased, diffuse | | | | | | |
| *Grades: Minimal* | 0 | 0 | 0 | 2 | 0 | 1 |
| *Mild* | 0 | 0 | 0 | 1 | 0 | 0 |
| *Moderate* | 0 | 0 | 0 | 1 | 0 | 0 |
| Oval cell hyperplasia | 0 (0.00) | 0 (0.00) | 0 (0.00) | 3 (60.0) | 0 (0.00) | 0 (0.00) |
| *Grades: Minimal* | 0 | 0 | 0 | 3 | 0 | 0 |
| Pericholangitis, (multi)focal | 0 (0.00) | 0 (0.00) | 0 (0.00) | 3 (60.0) | 0 (0.00) | 1 (20.0) |
| *Grades: Minimal* | 0 | 0 | 0 | 3 | 0 | 1 |

As a result of analyzing AST and ALT levels and examining histopathological findings, when the adenovirus was administered intravenously at a dose of 2.5×10¹⁰ VP/head, and then GCV was administered twice a day for 10 days, hepatocellular necrosis and inflammation occur, resulting in the induction of liver damage. However, when the adenovirus was administered alone at 0.25×10¹⁰ VP/head and 1.0×10¹⁰ VP/head or in combination with GCV, up to day 15 after the administration, liver damage-associated AST and ALT levels increased. However, according to the histological examination performed on day 29, a meaningful toxicological change which would be considered to be related to adenovirus administration was not observed in the liver.

### Comparative Example 1: Comparison of efficacy of CRT-122T and ECRT-122T

### 1-1. Comparison of anticancer efficacy

Anticancer efficacy was compared by inducing the generation of liver cancer by injecting 3×10⁶ Hep3B cells into an international standard mouse model and administering an adenovirus including a CRT-122T or ECRT-122T vector to the mouse model. The CRT-122T vector is a type of vector in which a SD/SA sequence and WPRE are removed from the ECRT-122T vector.

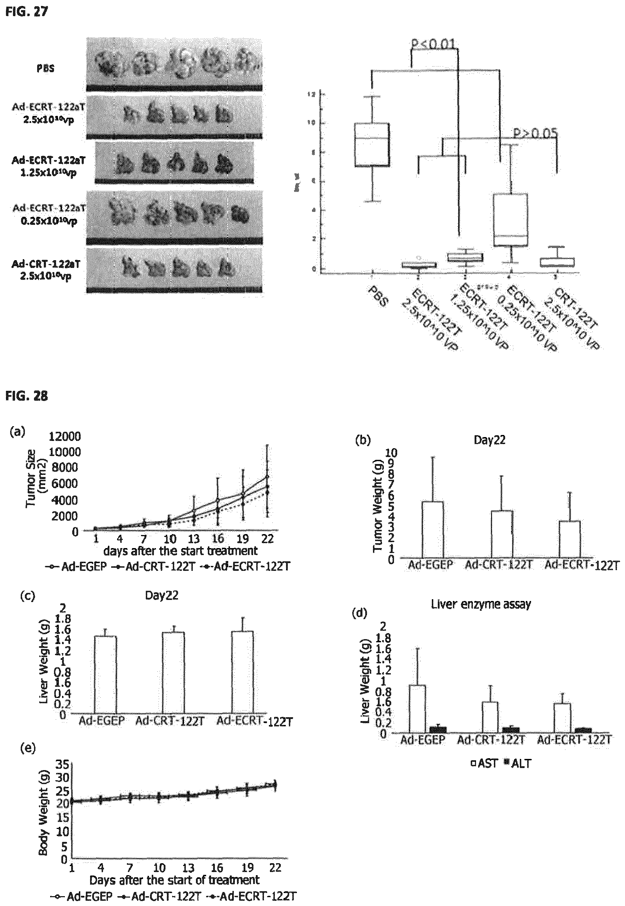

As a result, as shown in FIG. 27, it can be seen that when an adenovirus was injected at 2.5×10¹⁰ VP (virus particle), when compared with CRT-122T, ECRT-122T had superior anticancer efficacy. The anticancer efficacy of CRT-122T was similar to that of ECRT-122T being administered at a dose of 1.25x10¹⁰ VP.

### 1-2. Comparison of anticancer efficacy

Tumorigenesis was induced by injecting SNU398 cells into a mouse xenograft subcutaneous model, and when tumors had grown to a certain size or more, an adenovirus including a CRT-122T or ECRT-122T vector was intratumorally (I.T.) injected a total of two times at a dose of 1x10⁹ VP once every 2 days. After the adenovirus injection, while the mice were being observed, a tumor size and a body weight were measured every three days, and 22 days later, the mice were sacrificed to measure a final tumor size, a liver weight, and aspartate transaminase (AST) and alanine transaminase (ALT) levels.

As a result, as shown in FIGS. 28(a) and 28(b), it can be confirmed that compared with CRT-122T, ECRT-122T had superior anticancer efficacy. There was no significant difference in the body weight and liver weight of mice between experimental groups, and the AST and ALT levels indicated that the adenovirus did not induce hepatotoxicity (FIGS. 28(c) to 28(e)).

According to the above-described result, it was confirmed that a sufficient anticancer effect can be obtained by injecting only a small dose of the adenovirus, and compared with CRT-122T, it can be seen that ECRT-122T had significantly superior anticancer efficacy.

### 1-3. Comparison of toxicity

To confirm toxicity according to an injection dose of the adenovirus, the ECRT or ECRT-122T adenovirus was injected into normal ICR mice, and on days 2, 7 and 14 after injection, AST and ALT levels were measured.

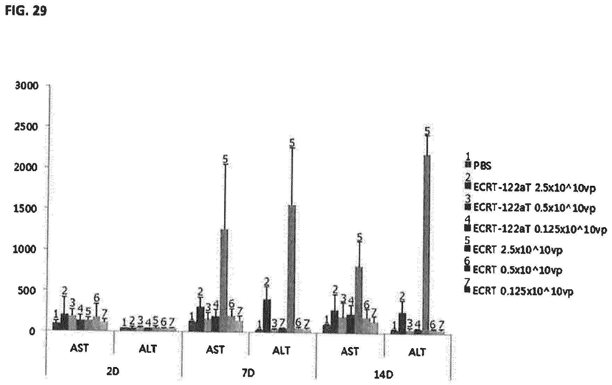

As a result, as shown in FIG. 29, in the mouse administered with ECRT at a dose of 2.5×10¹⁰ VP, very high hepatotoxicity was maintained until day 14 after the injection, but in the mouse administered with ECRT-122T at a dose of 2.5×10¹⁰ VP, hepatotoxicity was very low in comparison to the ECRT-administered mouse.

Since a trans-splicing ribozyme according to the present invention does not act on normal tissue, but is specifically expressed in cancer tissue, it is very safe and has excellent expression efficiency at the post-transcription level, and thus can be effectively used in treatment of cancer. Specifically, due to having a CMV promoter and SD/SA and WPRE sequences, a recombinant vector of the present invention can have high ribozyme expression efficiency, and due to miR-122T, the recombinant vector can regulate activity through miR-122 and thus have excellent safety. Therefore, the recombinant vector of the present invention can be effectively used in treatment of all types of liver cancer, except some types caused by HCV in which miR-122 expression in cancer tissue is highly shown in comparison to normal tissue, and the recombinant vector of the present invention also can be effectively used in treatment of other types of cancer in which miR-122 is not substantially expressed.

It will be apparent to those skilled in the art that various modifications can be made to the above-described exemplary embodiments of the present invention without departing from the spirit or scope of the invention. Thus, it is intended that the present invention covers all such modifications provided they come within the scope of the appended claims and their equivalents.

## Claims

1. A recombinant vector, comprising:
(i) a cytomegalovirus (CMV) promoter;
(ii) a ribozyme-desired gene expression cassette, which includes a sequence encoding a trans-splicing ribozyme targeting a cancer-specific gene and a target gene connected to a 3' exon of the ribozyme-encoding sequence,
wherein the vector has a splicing donor/splicing acceptor sequence (SD/SA sequence) connected to the 5' end of the ribozyme-desired gene expression cassette, and a Woodchuck hepatitis virus posttranscriptional regulatory element (WPRE) connected to the 3' end of the ribozyme-desired gene expression cassette; and
(iii) a nucleic acid sequence recognizing microRNA-122 (miR-122), which is connected to the 3' end of the WPRE.

2. The recombinant vector of claim 1, wherein the cancer-specific gene sequence is selected from the group consisting of a telomerase reverse transcriptase (TERT) mRNA sequence, an alphafetoprotein (AFP) mRNA sequence, a carcinoembryonic antigen (CEA) mRNA sequence, a prostate-specific antigen (PSA) mRNA sequence, a cytoskeleton-associated protein 2 (CKAP2) mRNA sequence, and a mutant rat sarcoma (RAS) mRNA sequence.

3. The recombinant vector of claim 2, wherein the TERT mRNA sequence comprises the nucleic acid sequence of SEQ ID NO: 2.

4. The recombinant vector of claim 1, wherein the trans-splicing ribozyme comprises the nucleic acid sequence of SEQ ID NO: 3.

5. The recombinant vector of claim 1, wherein the desired gene is an anti-cancer therapeutic gene or a reporter gene.

6. The recombinant vector of claim 5, wherein the anti-cancer therapeutic gene is selected from the group consisting of a drug-sensitizing gene, a proapoptotic gene, a cytostatic gene, a cytotoxic gene, a tumor suppressor gene, an antigenic gene, a cytokine gene and an anti-angiogenic gene.

7. The recombinant vector of claim 6, wherein the drug-sensitizing gene is a herpes simplex virus thymidine kinase (HSVtk) gene.

8. The recombinant vector of claim 7, wherein the HSVtk gene comprises the nucleic acid sequence of SEQ ID NO: 4.

9. The recombinant vector of claim 1, wherein the nucleic acid sequence recognizing miRNA-122 comprises one or more copies of a nucleic acid sequence of SEQ ID NO: 5.

10. A gene delivery system comprising the recombinant vector of claim 1.

11. A ribozyme expressed from the recombinant vector of claim 1.

12. A pharmaceutical composition for preventing or treating cancer, comprising the recombinant vector of claim 1, the gene delivery system of claim 10, or the ribozyme of claim 11 as an active ingredient.

13. The pharmaceutical composition of claim 12, wherein the cancer is liver cancer, glioblastoma, bile duct cancer, lung cancer, pancreatic cancer, melanoma, bone cancer, breast cancer, colon cancer, stomach cancer, prostate cancer, leukemia, uterine cancer, ovarian cancer, lymphoma, or brain cancer.

14. The pharmaceutical composition of claim 12, wherein with respect to the cancer, the copy number of miR-122 expressed in cancer tissue is less than 100 times the copy number of ribozymes expressed in cancer tissue due to the effect of a pharmaceutical composition.

15. The pharmaceutical composition of claim 14, wherein with respect to the cancer, miR-122 is not substantially expressed in cancer tissue.
